(19) Europäisches Patentamt
European Patent Office
Office européen des brevets

(11) EP 3 840 080 B1

(12) **EUROPEAN PATENT SPECIFICATION**

(45) Date of publication and mention of the grant of the patent:
**17.06.2026 Bulletin 2026/25**

(21) Application number: **19217137.9**

(22) Date of filing: **17.12.2019**

(51) International Patent Classification (IPC):
***H10K 85/60*** *(2023.01)* *H10K 50/16* *(2023.01)*

(52) Cooperative Patent Classification (CPC):
**H10K 85/654; H10K 85/6574;** H10K 50/165; H10K 50/19

(54) **ORGANIC ELECTRONIC DEVICE AND DISPLAY DEVICE COMPRISING THE ORGANIC ELECTRONIC DEVICE**

ORGANISCHE ELEKTRONISCHE VORRICHTUNG UND ANZEIGEVORRICHTUNG MIT DER ORGANISCHEN ELEKTRONISCHEN VORRICHTUNG

DISPOSITIF ÉLECTRONIQUE ORGANIQUE ET DISPOSITIF D'AFFICHAGE COMPRENANT LE DISPOSITIF ÉLECTRONIQUE ORGANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.06.2021 Bulletin 2021/25**

(73) Proprietor: **Novaled GmbH**
**01099 Dresden (DE)**

(72) Inventors:
- **GALAN GARCIA, Elena**
  **01099 Dresden (DE)**
- **SCHULZE, Benjamin**
  **01099 Dresden (DE)**

(74) Representative: **Michalski Hüttermann & Partner mbB**
**Kaistraße 16A**
**40221 Düsseldorf (DE)**

(56) References cited:
**WO-A1-2017/014546**
**CN-A- 110 229 145**
**KR-A- 20160 006 629**
**US-A1- 2016 260 901**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 840 080 B1

## Description

### Technical Field

The present invention relates to an organic semiconductor layer and an organic electronic device comprising the same. The invention further relates to a display device comprising the organic electronic device.

### Background Art

Organic electronic devices, such as organic light-emitting diodes OLEDs, which are selfemitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed. In particular the development of an organic semiconductor material or semiconductor layer is needed with respect to lowering the operating voltage, which is important for reducing power consumption and increasing battery life, for example of a mobile display device.

There remains a need to improve performance of organic semiconductor materials, organic semiconductor layers, as well as organic electronic devices thereof, in particular to achieve increased efficiency through improving the characteristics of the compounds comprised therein.

Substituted pyrazines are *inter alia* disclosed in the CN 110 229 145 A, KR 2016 0006629 A and WO 2017/145546 A1.

### DISCLOSURE

An aspect of the present invention provides an organic electronic device comprising an anode, a cathode, at least one photoactive layer and at least one organic semiconductor layer, wherein the at least one organic semiconductor layer is arranged between the at least one photoactive layer and the cathode; and wherein the at least one organic semiconductor layer comprises a $C_s$-symmetric compound of Formula (1):

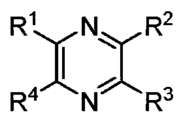

(1)

wherein

- $R^1$-$R^4$ are independently selected from a substituted or unsubstituted $C_6$-$C_{36}$ aryl, and a substituted or unsubstituted $C_3$-$C_{36}$ heteroaryl;
- wherein at least one of the $R^1$-$R^4$ is selected from $C_3$-$C_{36}$ heteroaryl group or heteroarylene group which is directly attached to the pyrazine ring in formula 1 and at least three of $R^1$-$R^4$ are different to each other;
- wherein, if substituent(s) are present in $R^1$, $R^2$, $R^3$, and $R^4$, the substituent(s) are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, (C=O)$N(R)_2$, $Si(R)_3$, (S=O)R, and

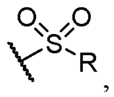

whereby

Y is O or S; and

R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

wherein two of the $R^1$-$R^4$ are phenyl or naphthenyl;

and wherein the following compounds 1-4 are excluded:

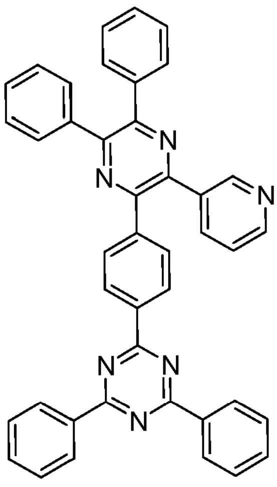
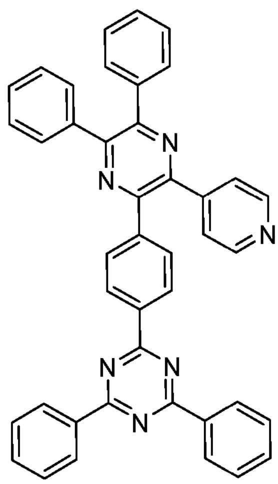
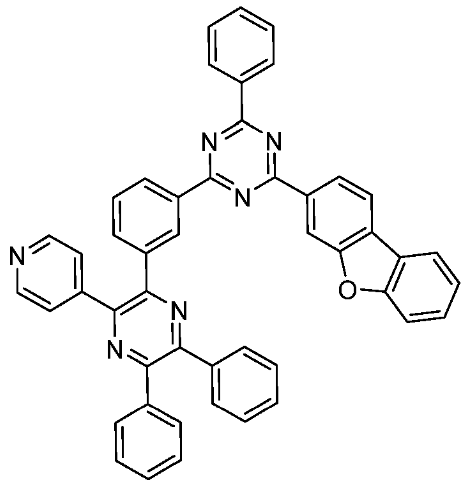

**1** , 2 , **3** ,

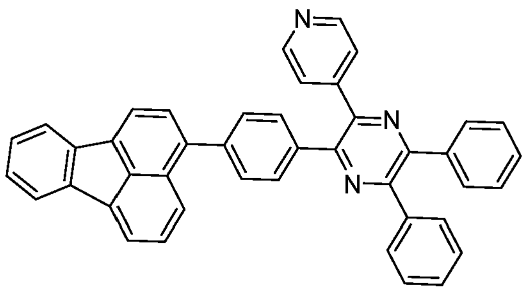

**4** .

Another aspect of the present invention provides A $C_s$-symmetric compound of Formula (1):

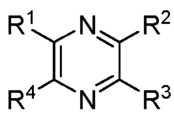

(1)

wherein

$R^1$-$R^4$ are independently selected from a substituted or unsubstituted $C_6$-$C_{36}$ aryl, and a substituted or unsubstituted $C_3$-$C_{36}$ heteroaryl;

wherein at least one of the $R^1$-$R^4$ is selected from $C_3$-$C_{36}$ heteroaryl group or heteroarylene group which is directly attached to the pyrazine ring in formula 1 and at least three of $R^1$-$R^4$ are different to each other;

wherein, if substituent(s) are present in $R^1$, $R^2$, $R^3$, and $R^4$, the substituent(s) are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

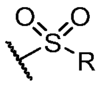
,

whereby
Y is O or S; and
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;
wherein two of the $R^1$-$R^4$ are phenyl or naphthenyl;
wherein at least one of the $R^1$ to $R^4$ is selected from formula (2):

$$-L-(Ar)_n \qquad (2)$$

whereby
L is substituted or unsubstituted $C_6$-$C_{36}$ arylene
Ar is selected from a substituted or unsubstituted $C_6$-$C_{36}$ aryl, and a substituted or unsubstituted $C_3$-$C_{36}$ heteroaryl;
n is 1 to 5;
two or more groups Ar may be identical to or different from each other;
and wherein the following compounds are excluded:

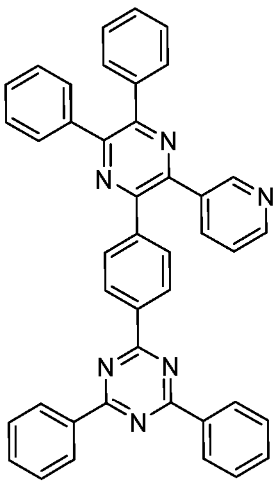
**1** ,

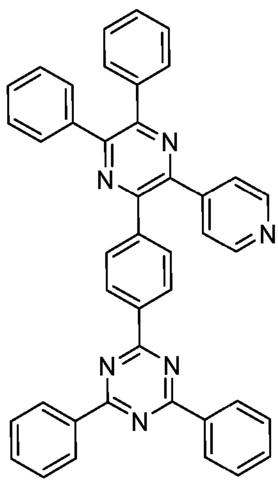
2 ,

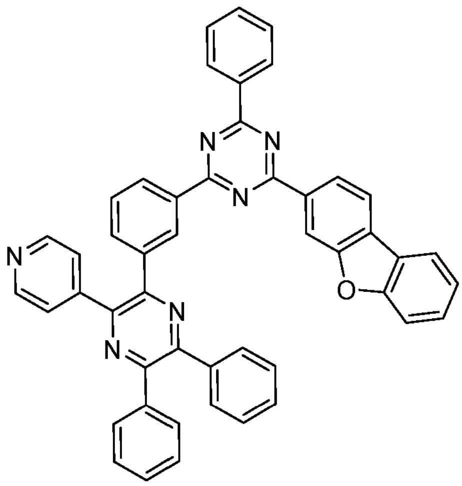
**3** ,

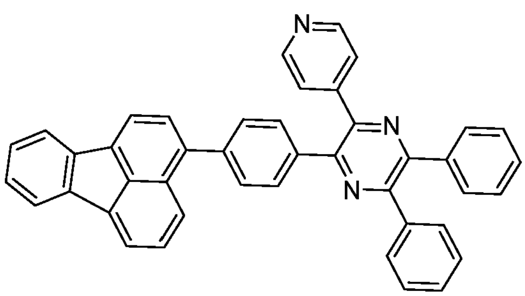
**4** .

It should be noted that throughout the application and the claims any abbreviations for chemical moietis such as $R^n$, X or Y always refer to the same moieties, unless otherwise noted.

The term "$C_s$-symmetric" especially means and/or includes that the symmetry of the compound according to formula (1) belongs to the $C_s$-Point group, which means that the only two symmetry operations that are available are E (= identity) and σ (= mirror plane). For further explanations it is referred to the description on the website of the Ludwig-Maximilians-Universität München (cf: https://www.cup.uni-muenchen.de/ch/compchem/geom/sym_Cs.html)

In the present specification, when a definition is not otherwise provided, "substituted" refers to $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

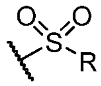,

whereby Y is O or S and R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl

However, in the present specification especially "aryl substituted" refers to a substitution with one or more aryl groups, which themselves may be substituted with one or more aryl and/or heteroaryl groups.

Correspondingly, in the present specification "heteroaryl substituted" especially refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenylyl, and polycyclic groups comprising fused rings, like naphtyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electron formed in the cathode may be easily injected into the emission layer and transported in the emission layer due to conductive characteristics according to a lowest unoccupied molecular orbital (LUMO) level.

## Advantageous Effects

Surprisingly, it was found that the organic electronic device according to the invention solves the problem underlying the present invention by enabling devices in various aspects superior over the organic electroluminescent devices known in the art, in particular with respect to efficiency, operating voltage and/or lifetime.

According to one embodiment of the present invention, in the compound of formula (1) the $R^1$ to $R^4$ that are not a substituted or unsubstituted $C_3$-$C_{36}$ heteroaryl or heteroarylene, which is directly attached to the pyrazine ring in formula (1) through a single bond are selected from substituted or unsubstituted $C_6$-$C_{20}$ aryl, preferably substituted or unsubstituted $C_6$-$C_{24}$ aryl and a substituted or unsubstituted $C_3$-$C_{18}$ heteroaryl, preferably substituted or unsubstituted $C_3$-$C_{12}$ heteroaryl.

According to one embodiment of the present invention, in the compound of formula (1) the at least one of the $R^1$ to $R^4$ that is a substituted or unsubstituted $C_3$-$C_{36}$ heteroaryl or heteroarylene is a substituted or unsubstituted $C_3$-$C_{18}$ heteroaryl or arylene, preferably substituted or unsubstituted $C_3$-$C_{12}$ heteroaryl or heteroarylene.

According to one embodiment of the present invention the compound of formula (1) has a dipole moment of $\geq 0.4$ D, preferably $\geq 0.5$ D.

According to the present invention, at least three of $R^1$-$R^4$ in formula (1) are selected different from each other.

According to the present invention, two of the $R^1$-$R^4$ are phenyl or naphthenyl, preferably phenyl. More preferred, two of the $R^1$-$R^4$ are phenyl.

According to one embodiment of the present invention, two of the $R^1$-$R^4$ are phenyl or naphthenyl and in ortho position to each other.

According to one embodiment of the present invention, two of the $R^1$-$R^4$ are phenyl and in ortho position to each other.

According to one embodiment of the present invention, at least one of the $R^1$ to $R^4$ has the structure

$$-L-(Ar)_n \qquad (2)$$

whereby

L is substituted or unsubstituted $C_6$-$C_{36}$ arylene
Ar is selected from a substituted or unsubstituted $C_6$-$C_{36}$ aryl, and a substituted or unsubstituted $C_3$-$C_{36}$ heteroaryl;
n is 1 to 5;
two or more groups Ar may be identical to or different from each other;

According to one embodiment of the present invention, n is 1 or 2, preferably 1.

According to one embodiment of the present invention, L is selected from phenylene, biphenlyene, triphenlyene and naphthylene.

According to one embodiment of the present invention, L is chosen from one of the following moieties A1 to A16:

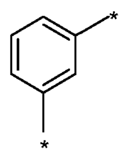

A1

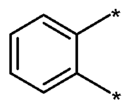

A2

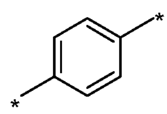

A3

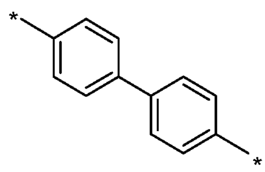

A4

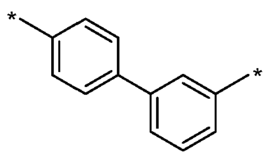

A5

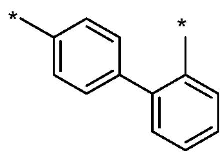

A6

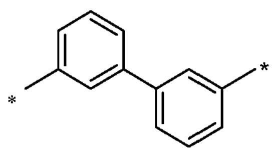

A7

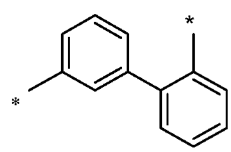

A8

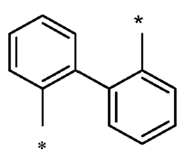 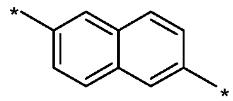 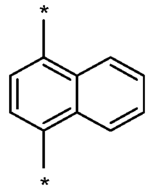 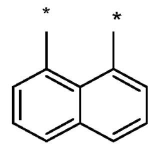

A9 A10 A11 A12

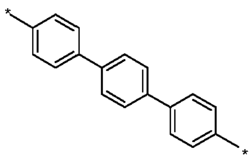 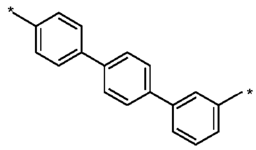 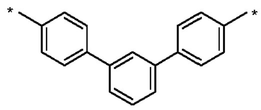 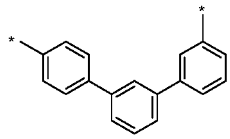

A13 A14 A15 A16.

wherein the asterisk symbol "*" represents the binding positions of L.

According to one embodiment of the present invention, at least one of $R^1$ to $R^4$ or Ar are a substituted or unsubstituted heteroaryl comprising a single five- or six-membered aromatic ring, or a substituted or unsubstituted heteroaryl comprising two condensed five- and/or six-membered aromatic rings, or a substituted or unsubstituted heteroaryl comprising three condensed fiveand/or six-membered aromatic rings; or a substituted or unsubstituted heteroaryl comprising more than three condensed five- and/or six-membered aromatic rings; whereby the heteroaryl contains 1-4 hetero-atoms selected from N, O and S.

According to one embodiment of the present invention, at least one of $R^1$ to $R^4$ or Ar may be selected from pyridine, quinolone, isoquinolone, indole, acridine, benzoacridine, dibenzoacridines, phenanthrodine, carbazole, indole, benzoindole, pyrimidine, pyrazine, quinozoline, pyrazole, quinoxaline, phenazine, naphthyridine, phananthrodine, azacarbazole, benzimidazole, benzooxazole, benzothiazole, nezotriazole, benzooxadiazole, benzothiadiazole, benzothiphene, benzofurane, dibenzofurane, dibenzothiophene, naphthofurane, naphthothiophene, phenanthroline.

According to one embodiment of the present invention, at least one of $R^1$ to $R^4$ or Ar are chosen from the following groups B1 to B27:

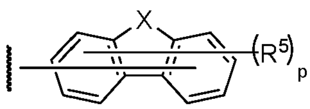

(B1)

whereby

X is $NR^6$, O or S,

$R^5$ and $R^6$ are independently selected from H, $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, (C=O)$N(R)_2$, $Si(R)_3$, (S=O)R, and

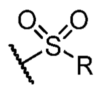

;

Y is O or S;

R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, C3-C20 cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

p is 0 to 7;

two or more groups $R^5$ may be identical to or different from each other.

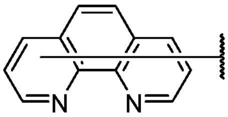 (B2), 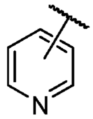 (B3), 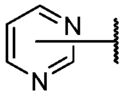 (B4), 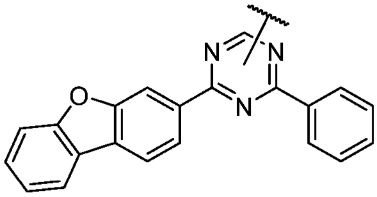 (B5),

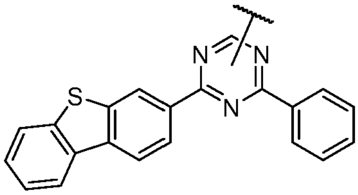 (B6), 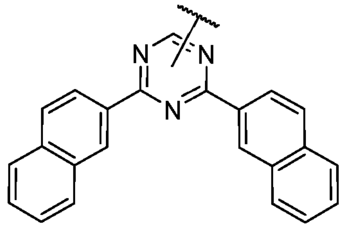 (B7),

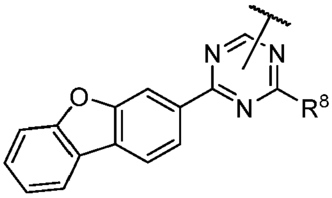 (B8),

wherein $R^8$ = naphthyl, *p*-biphenyl or *o*-biphenyl,

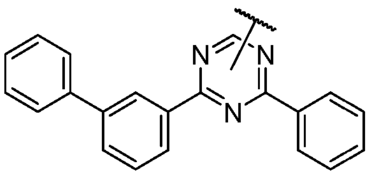 (B9), 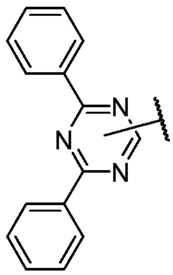 (B10),

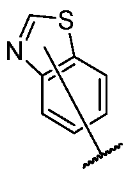 (B11), 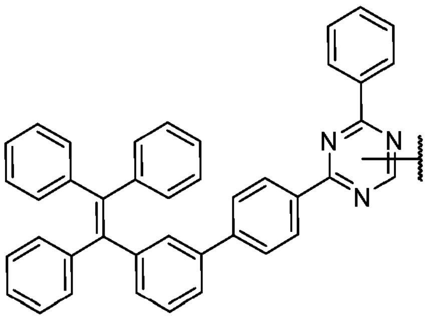 (B12),

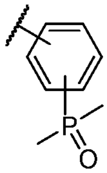 (B13), 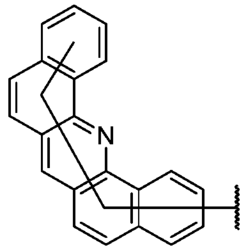 (B14), 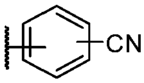 (B15),

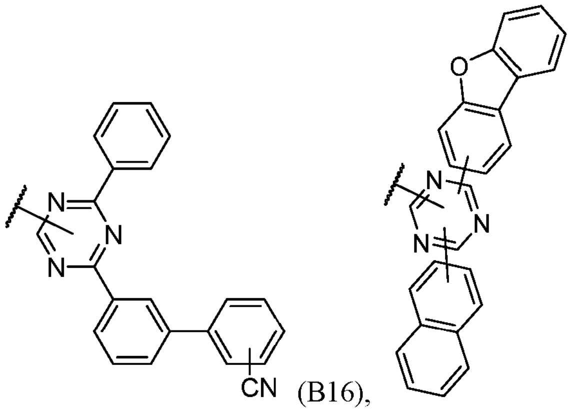 (B16), (B17),

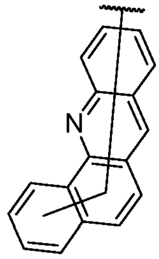 (B18), 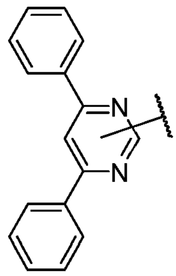 (B19), 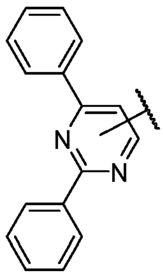 (B20),

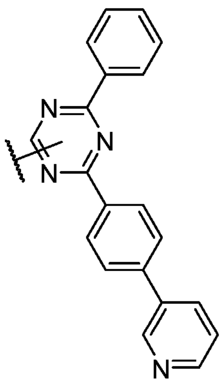 (B21), 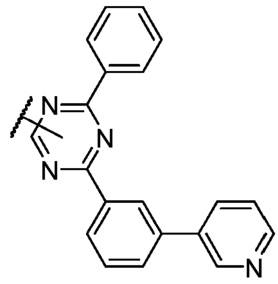 (B22),

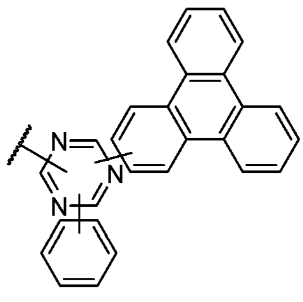 (B23), 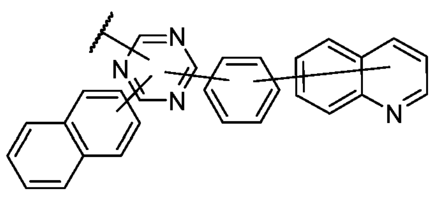 (B24),

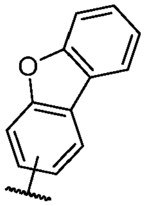 (B25), 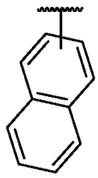 (B26), 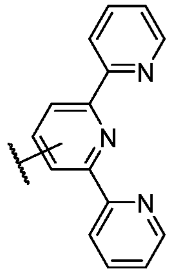 (B27).

According to one embodiment of the present invention, at least one of $R^1$ to $R^4$ or Ar are chosen from the following groups C1 to C15:

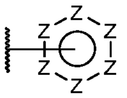 (C1), 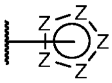 (C2), 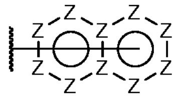 (C3), 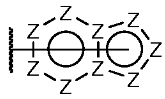 (C4), 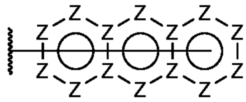 (C5),

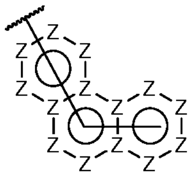 (C6), 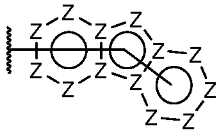 (C7), 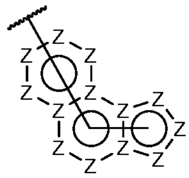 (C8), 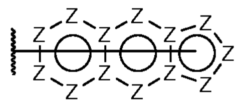 (C9),

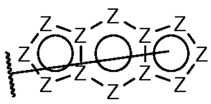 (C10), 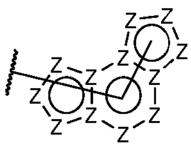 (C11), 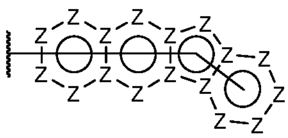 (C12),

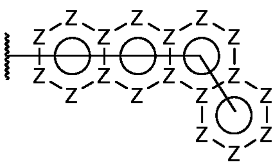 (C13), 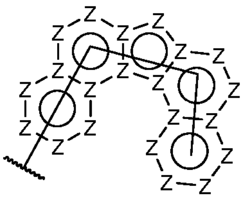 (C14), 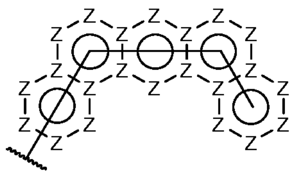 (C15);

wherein

Z is independently selected from C, CH, CR', N, NH, NR', O and S;
in each of the formulas C1-C15, at least one of Z is selected from N, NH, NR', O and S;
R' is independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, (C=O)N(R)$_2$, Si(R)$_3$, (S=O)R, and

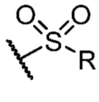 ;

Y is O or S;
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;
two or more groups R in each formula may be identical to or different from each other.

According to one embodiment of the present invention, heteroaryl comprising a single aromatic ring is selected

from pyridine, pyrimidine, pyrazine, triazine, pyrrole, furan, thiophene, imidazole, oxazole, thiazole, pyrazole, isoxazole, isothiazole, triazole.

According to one embodiment of the present invention, the heteroaryl comprising two condensed aromatic rings is selected from quinolone, isoquinoline, quinazoline, quinoxaline, naphthyridine, indole, isoindole, indolizine, benzofuran, isobenzofuran, benzothiophene, benzimidazole, benzoxazole, benzothiazole, indazole, benzisoxazole, benzisothiazole, benzotriazole, benzoxadiazole, benzothiadiazole.

According to one embodiment of the present invention, the heteroaryl comprising three condensed aromatic rings is selected from acridine, benzoquinoline, benzoisoquinoline, phenazine, phenanthridine, benzoquinoline, benzoisoquinoline, phenanthroline, carbazole, dibenzofuran, dibenzothiophene, azacarbazole, azadibenzofuran, azadibenzothiophene, benzoindole, naphtofuran, naphtothiophene, benzoindole, naphthofuran, naphthothiophene, benzodifuran, benzodipyrrole, benzodithiophene

According to one embodiment of the present invention, the heteroaryl comprising more than three condensed aromatic rings is selected from benzocarbazole, naphtobenzofuran, benzonaphthothiophene, dibenzocarbazole, dinaphthofuran, dinaphthothiophene, benzoacridine, benzophenanthroline, dibenzoacridine

According to one embodiment of the present invention, at least one, preferably exactly one $R^1$ to $R^4$ is B1.

According to one embodiment of the present invention, L is p-phenyl and Ar is either substituted or unsubstituted pyridinyl, phenyl or biphenyl.

According to one embodiment of the present invention, the compound is chosen from one of the following compounds I-1 to I-105

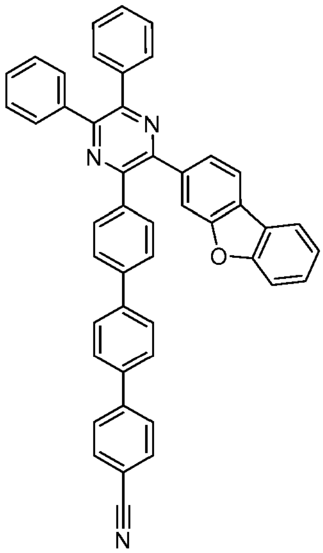

I-1

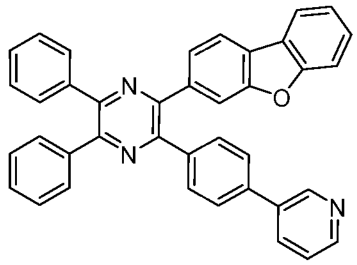

I-2

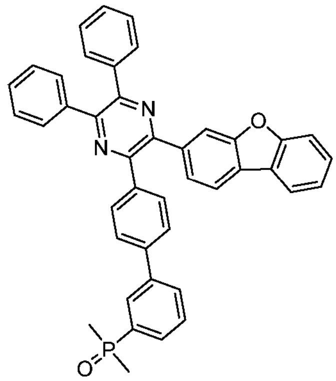

I-
3

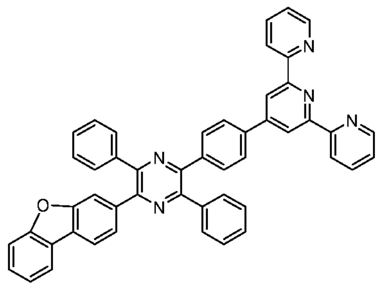

I-4

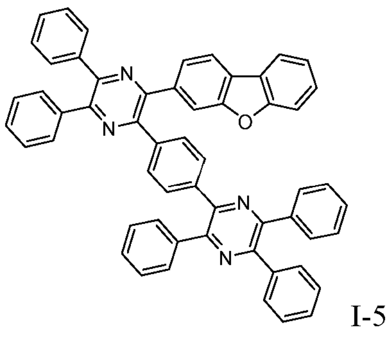

I-5

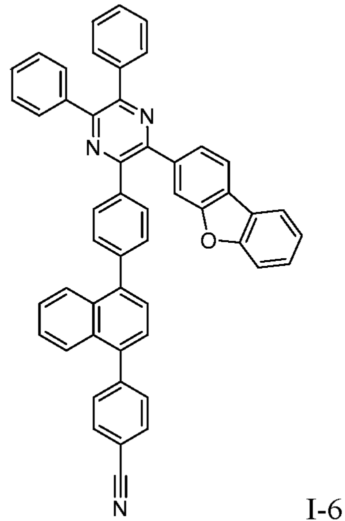

I-6

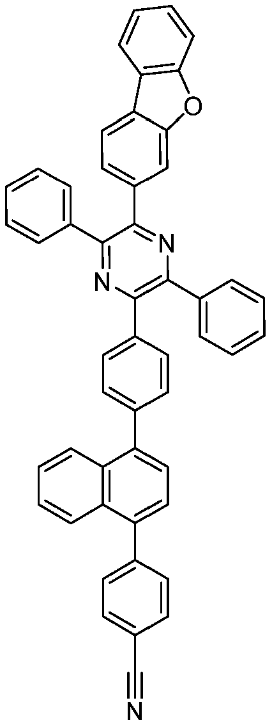

I-7

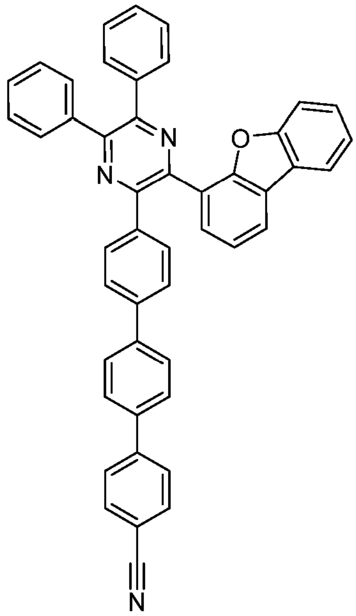

I-8

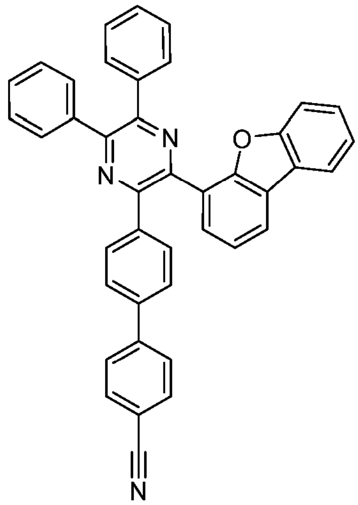

I-9

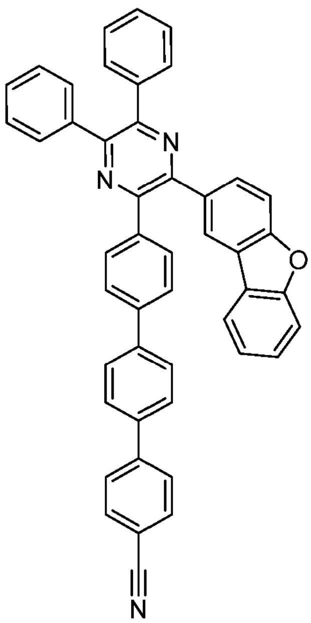

I-10

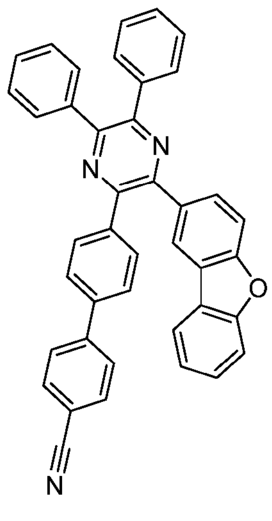

I-11

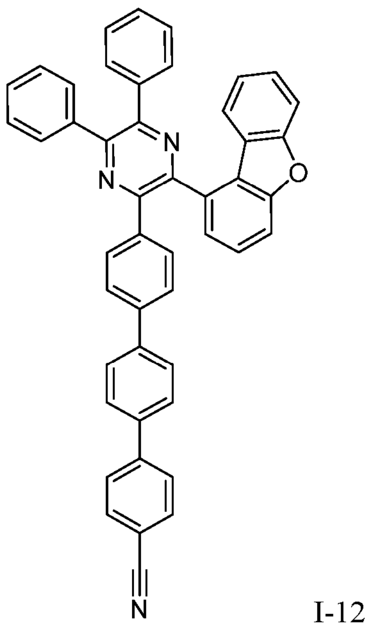

I-12

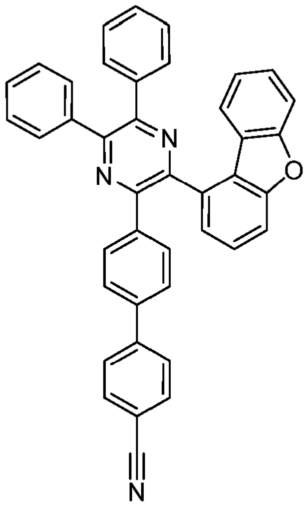
I-13

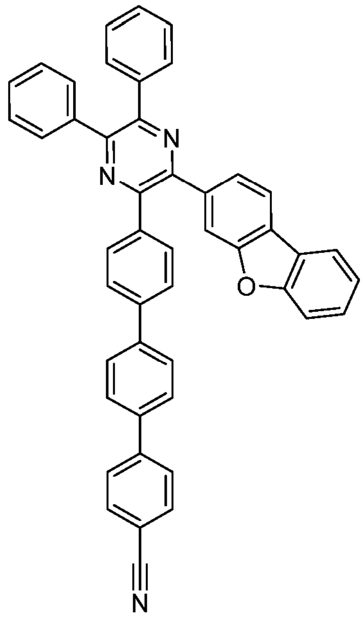
I-14

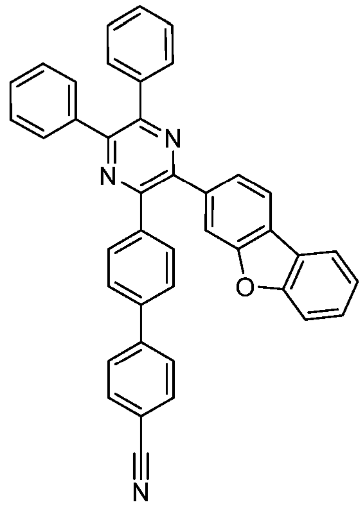
I-15

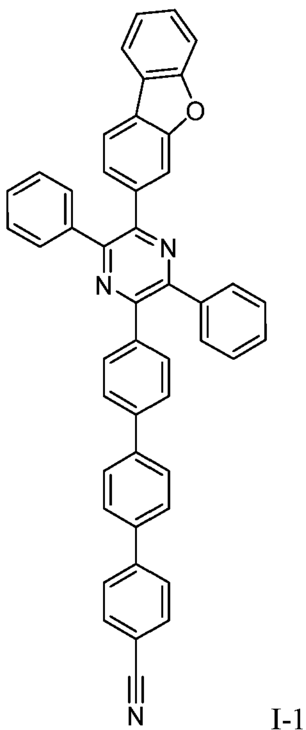
I-16

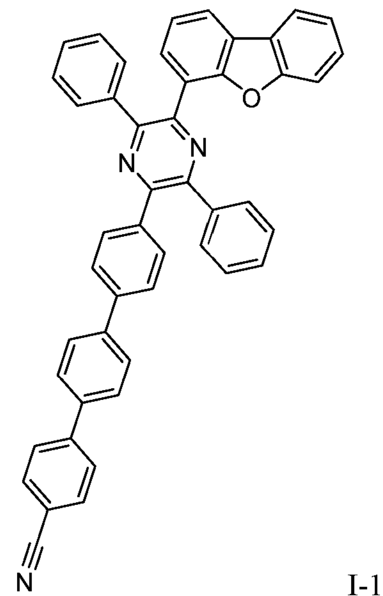
I-17

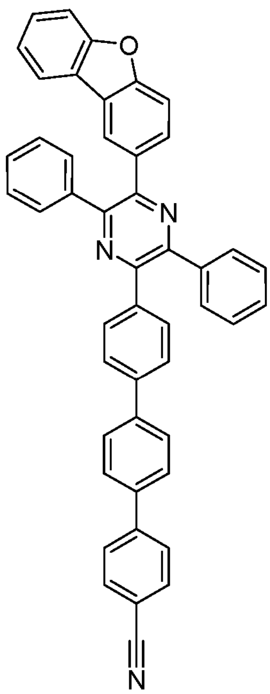
I-18

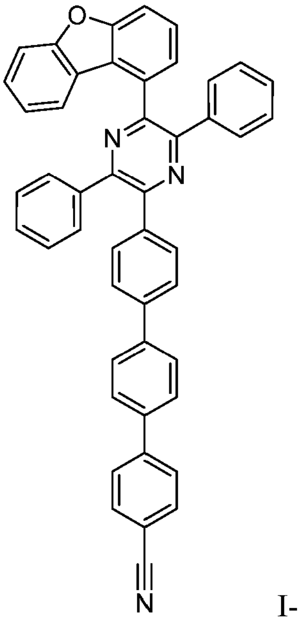

I-19

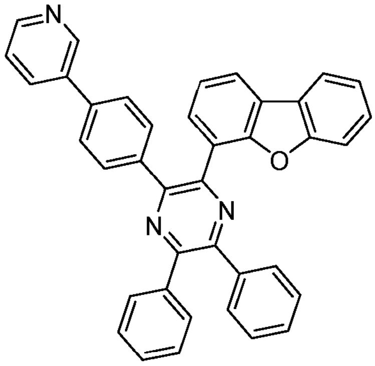

I-20

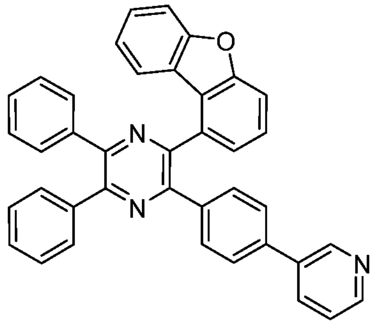

I-21

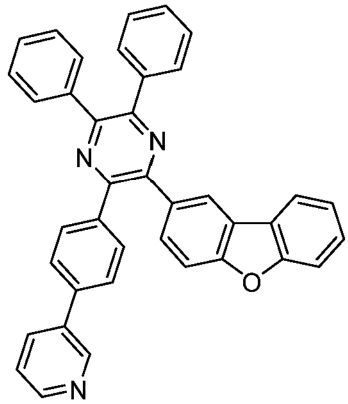

I-22

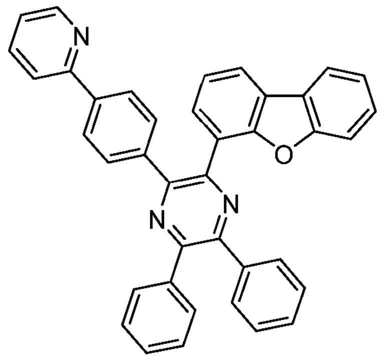

I-23

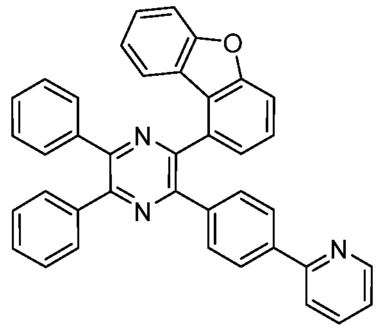

I-24

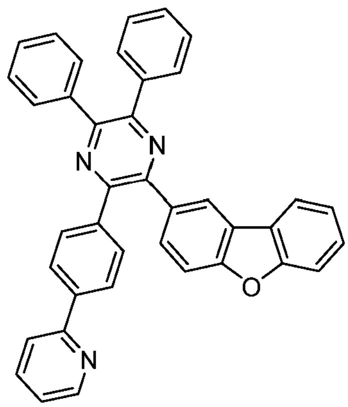

I-25

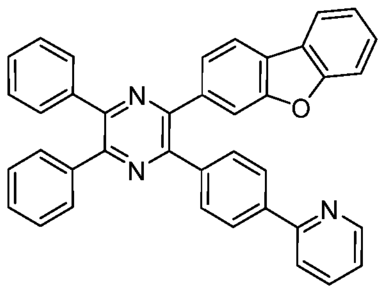

I-26

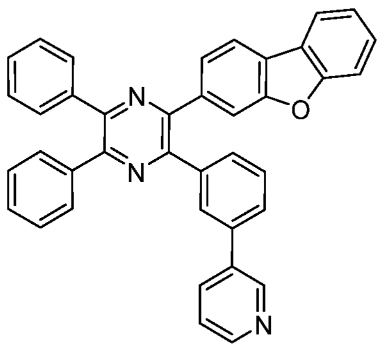

I-27

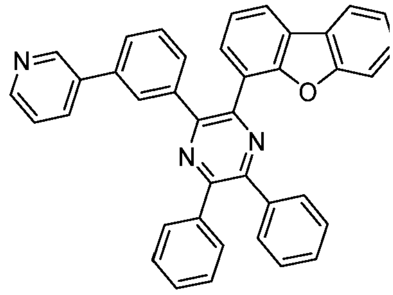
I-28

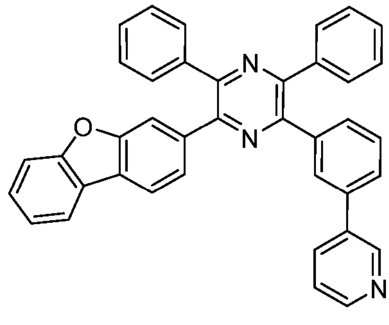
I-29

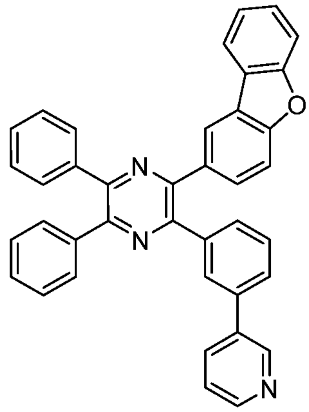
I-30

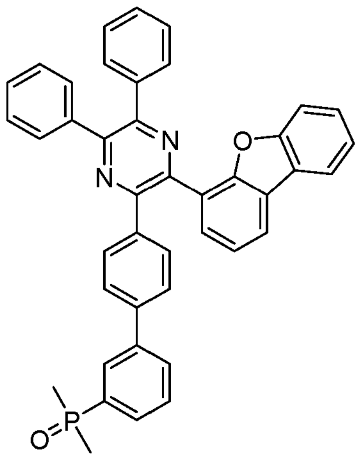
I-31

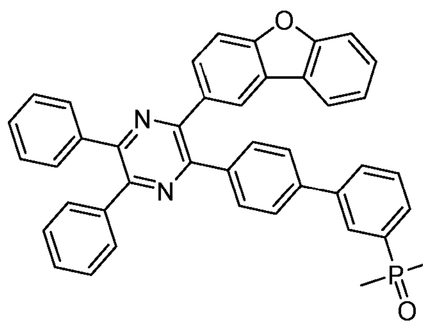
I-32

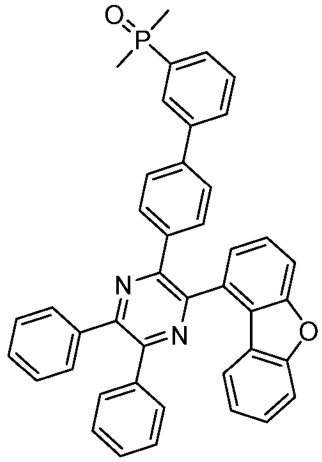
I-33

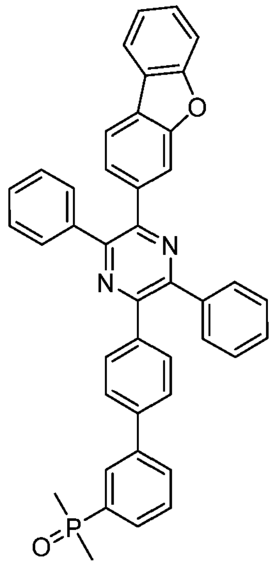
I-34

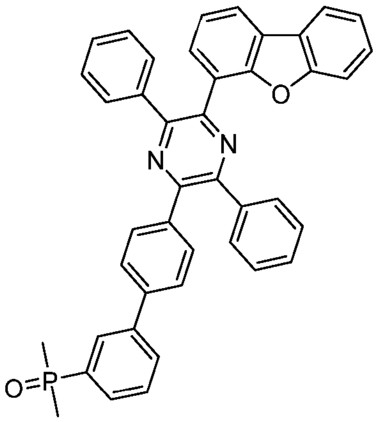
I-35

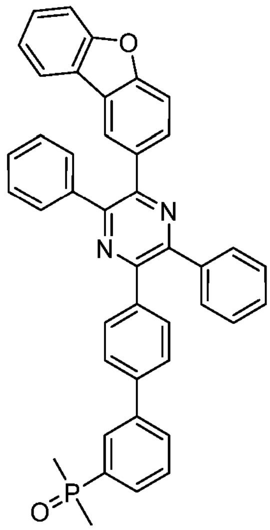
I-36

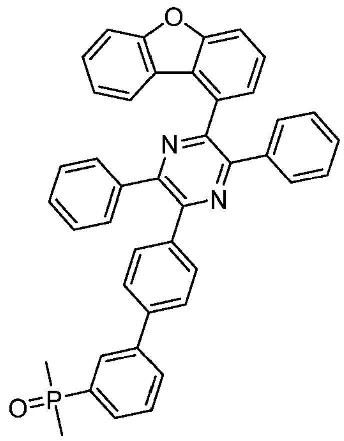

I-37

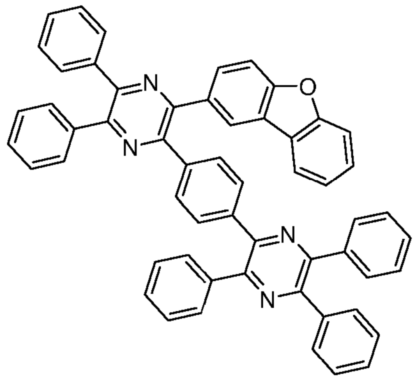

I-38

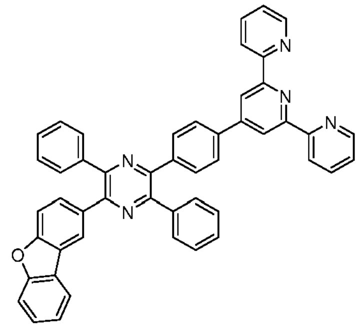

I-39

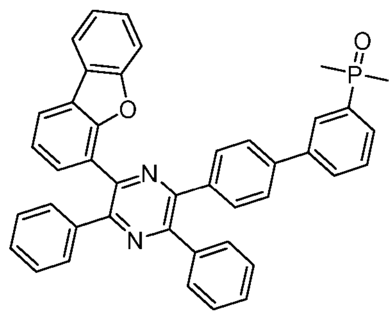

I-40

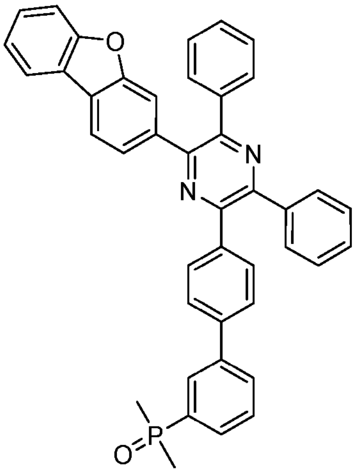

I-41

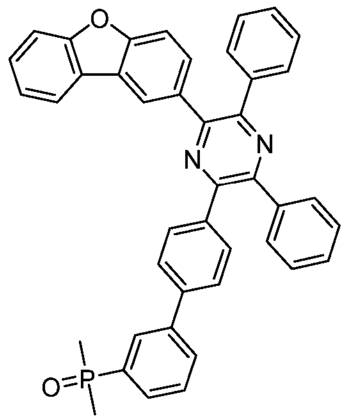

I-42

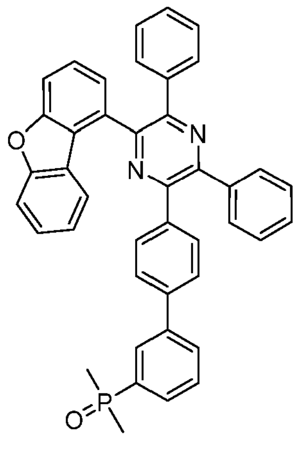

I-43

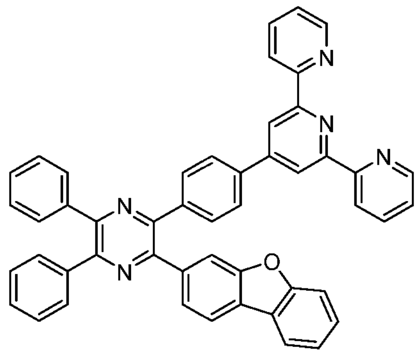

I-44

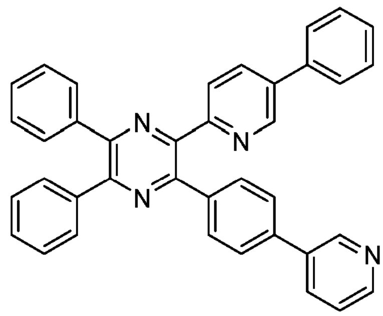

I-45

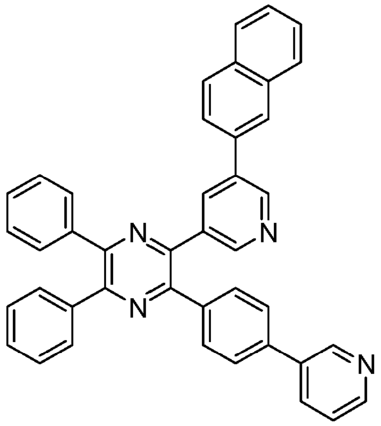

I-46

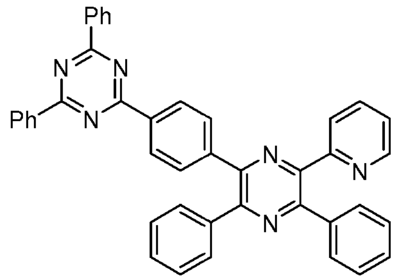

I-47

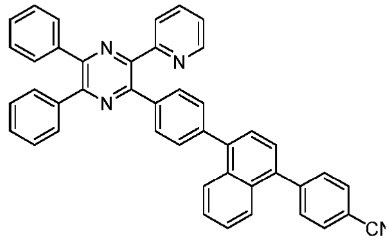

I-48

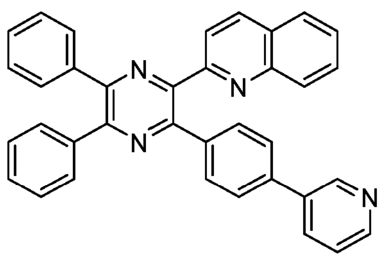

I-49

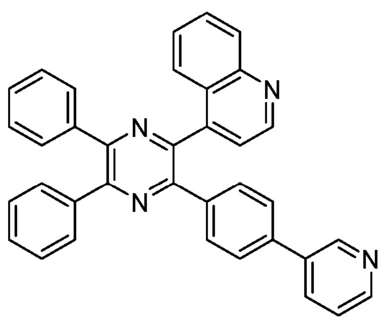

I-50

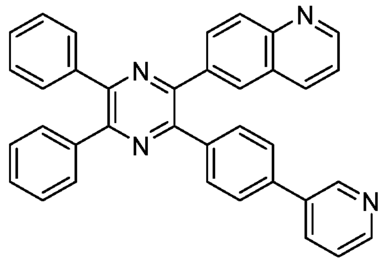

I-51

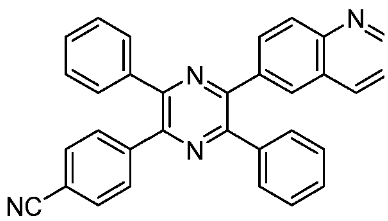

I-52

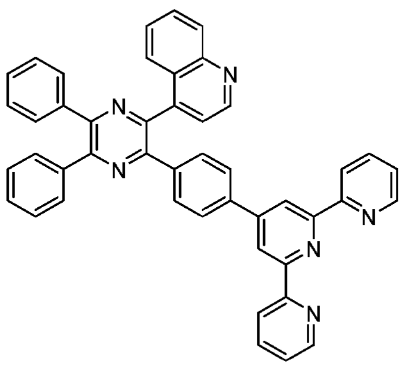

I-53

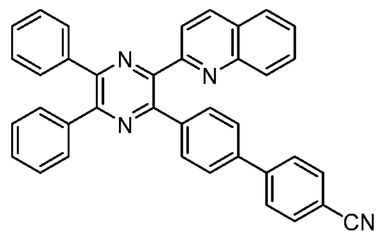

I-54

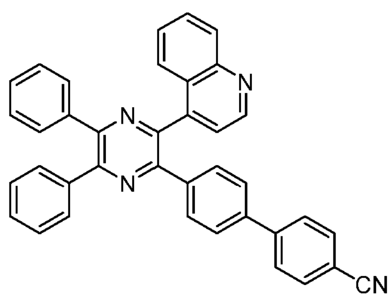

I-55

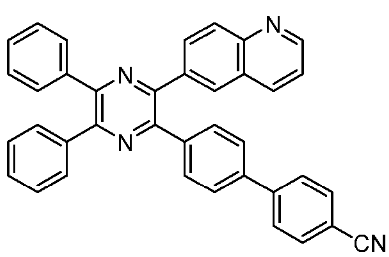

I-56

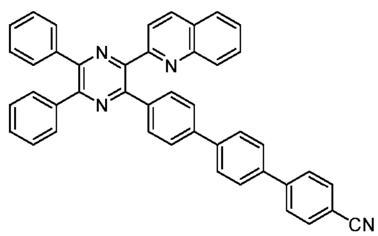

I-57

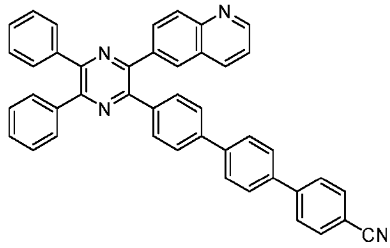

I-58

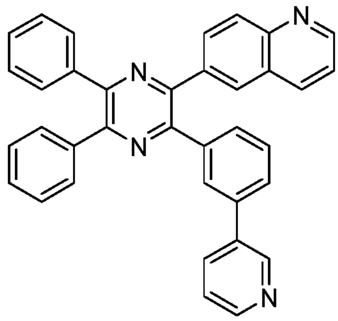

I-59

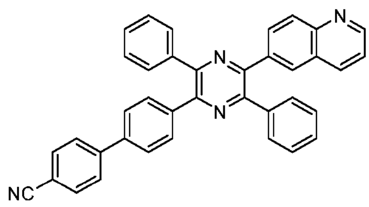

I-60

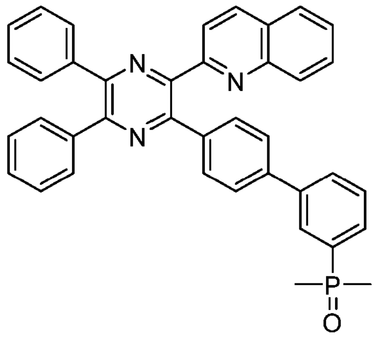

I-61

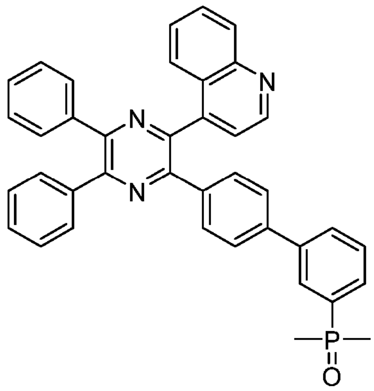

I-62

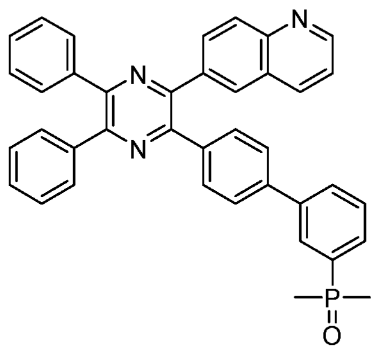

I-63

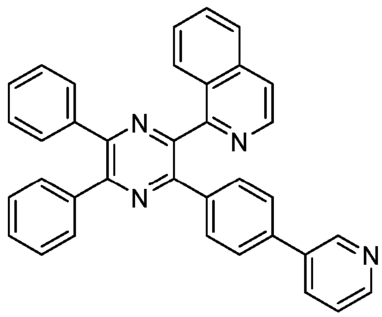

I-64

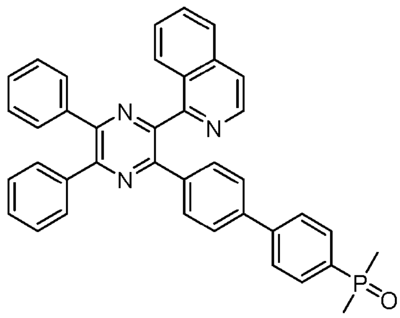

I-65

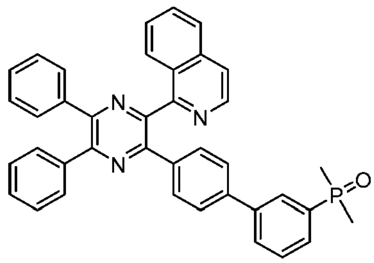

I-66

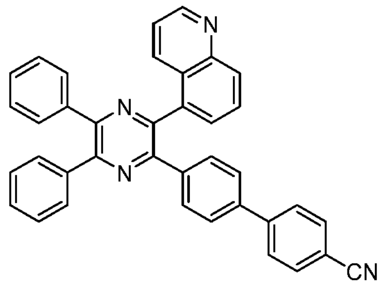

I-67

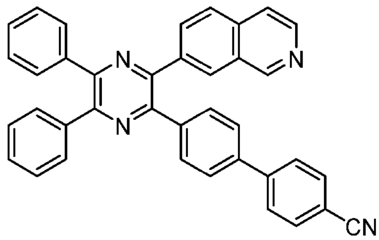

I-68

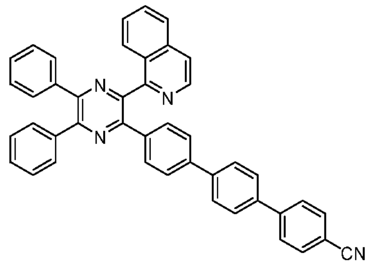

I-69

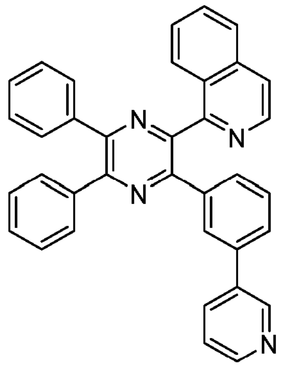

I-70

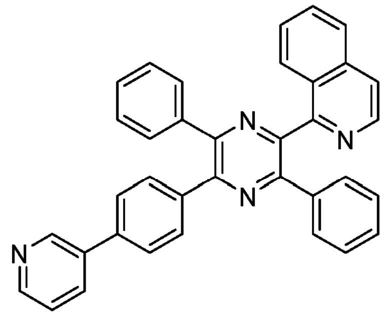

I-71

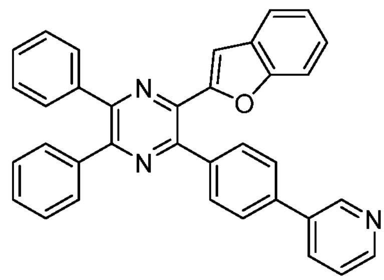

I-72

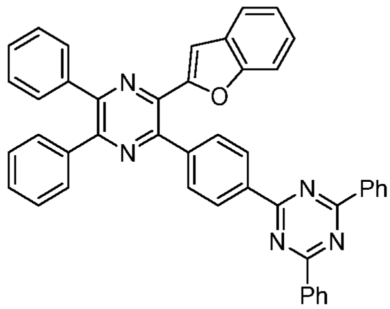

I-73

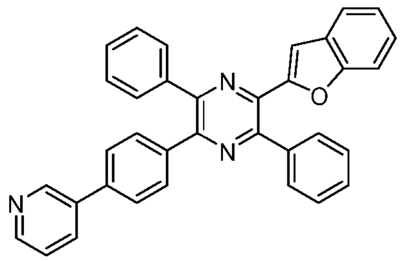

I-74

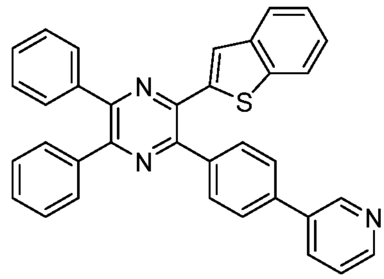

I-75

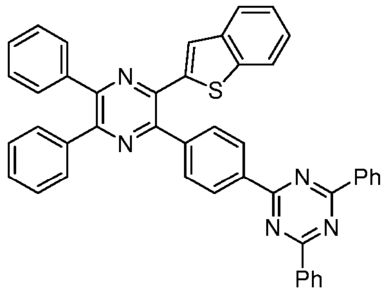

I-76

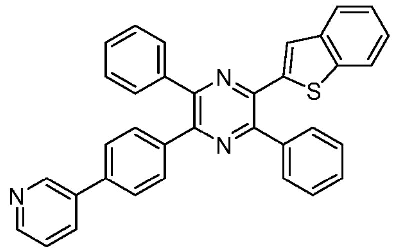

I-77

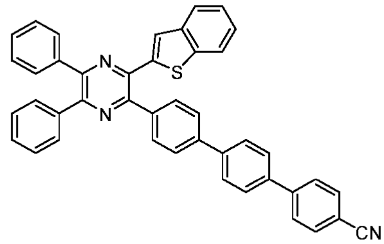

I-78

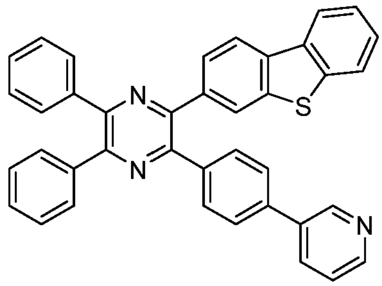

I-79

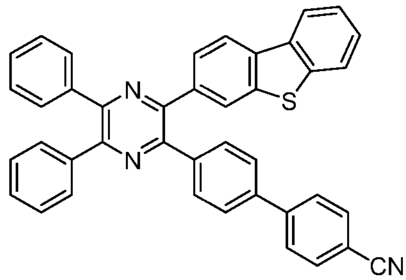

I-80

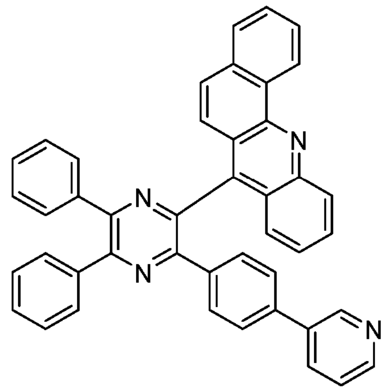

I-81

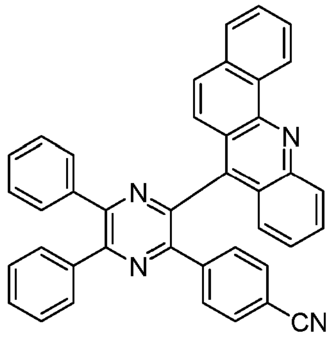

I-82

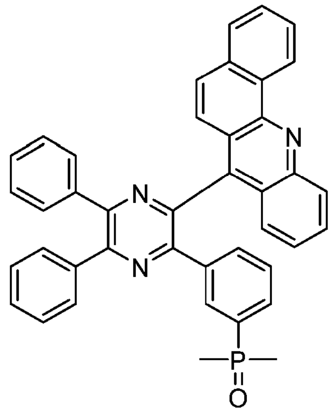

I-83

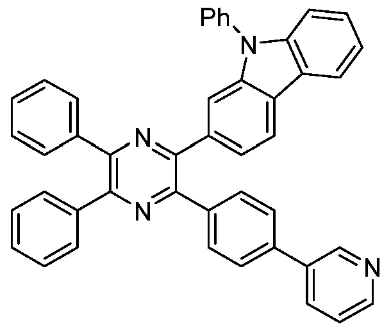

I-84

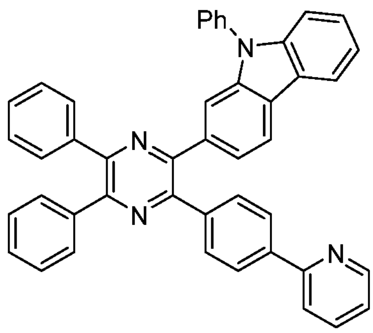

I-85

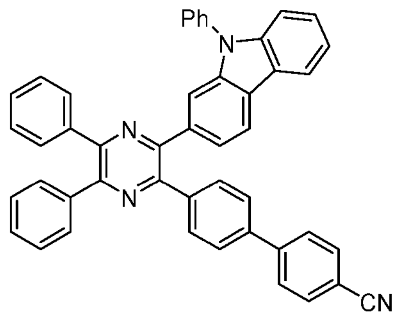

I-86

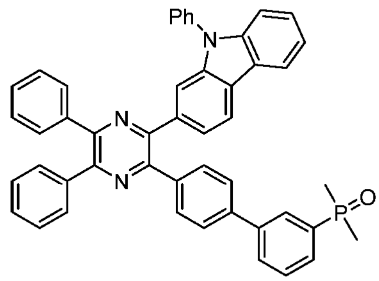

I-87

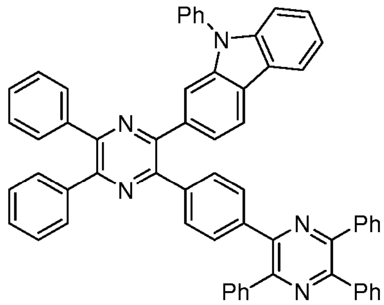

I-88

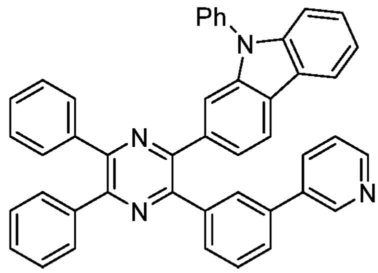

I-89

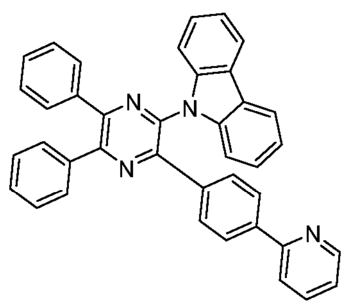

I-90

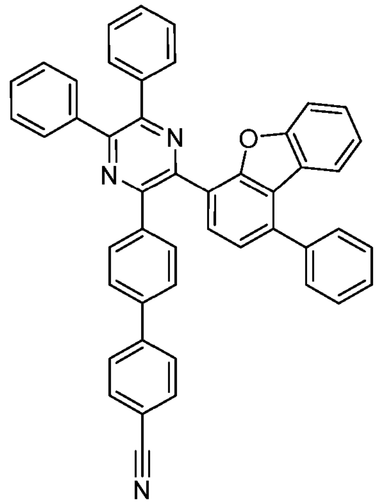

I-91

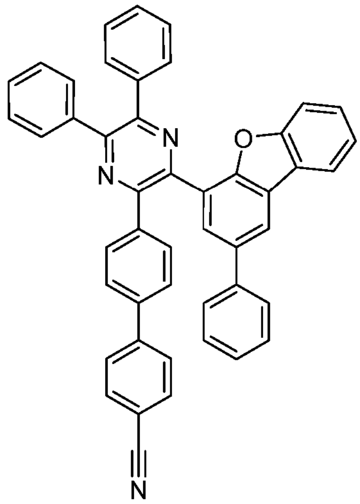

I-92

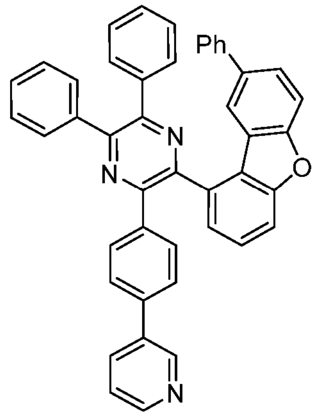

I-93

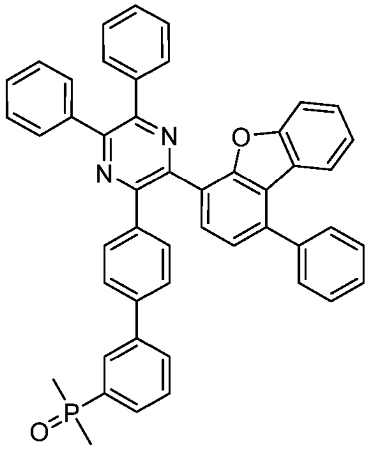

I-94

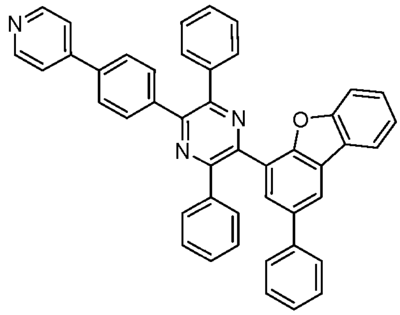

I-95

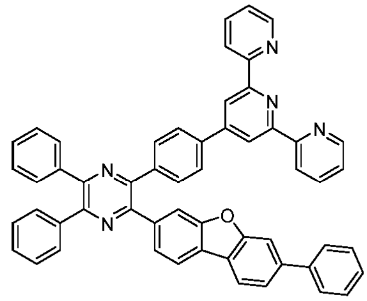

I-96

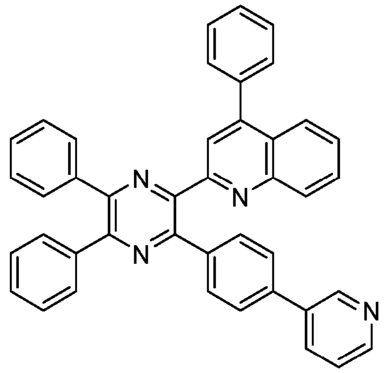

I-97

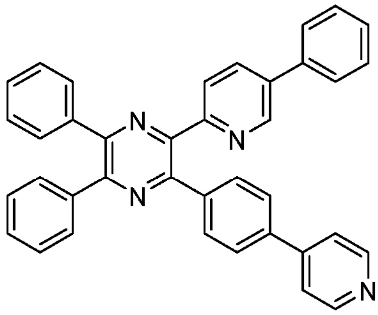

I-98

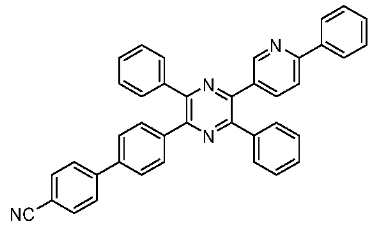

I-99

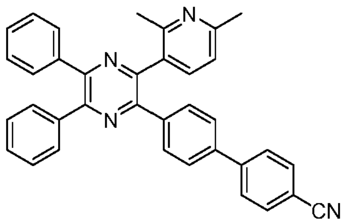

I-100

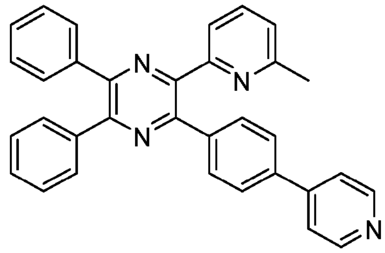

I.101

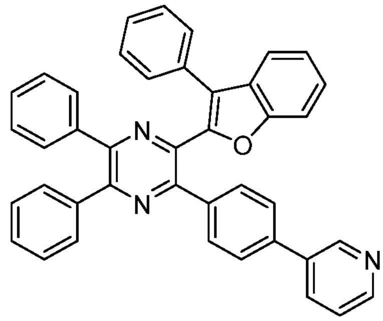

I-102

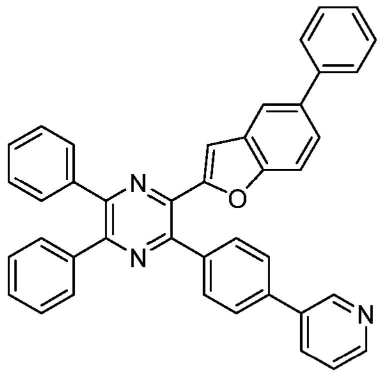

I-103

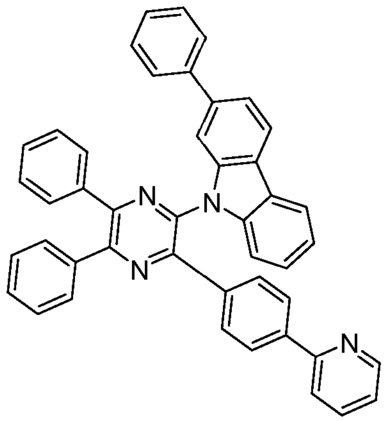

I-104

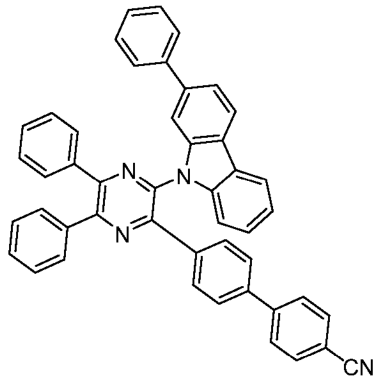

I-105

According to one embodiment of the present invention the organic semiconductor layer of the organic electronic device comprises a redox n-dopant.

Under redox n-dopant, it is understood a compound which, if embedded into an electron transport matrix, improves, in comparison with the neat matrix under the same physical condictions, the electron properties of the formed organic material, particularly in terms of electron injection and/or electron conductivity.

In the context of the present invention "embedded into an electron transport matrix" means homogenously mixed with the electron transport matrix.

The redox n-dopant may be selected from elemental metals, metal salts, metal complexes and organic radicals.

In one embodiment, the redox n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

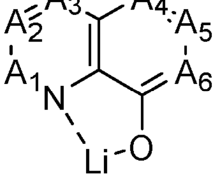 (II), 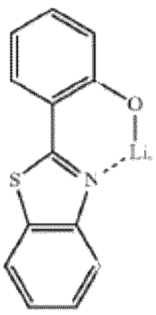 (III), 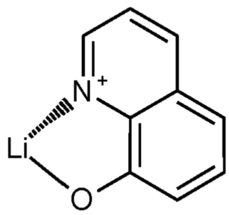 (IV)

wherein

A1 to A6 are same or independently selected from CH, CR, N, O;

R is same or independently selected from hydrogen, halogen, alkyl or aryl or heteroaryl with 1 to 20 carbon atoms; and more preferred A1 to A6 are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

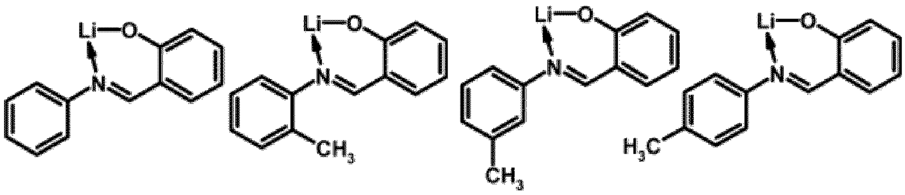

100 101 102 103

In another embodiment, the redox n-dopant is a redox n-dopant.

According to one embodiment of the invention, the organic semiconductor layer of the present invention comprises a lithium organic complex, alternatively LiQ.

According to one embodiment of the present invention the organic semiconductor layer of the organic electronic device comprises a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb

According to one embodiment of the invention, the organic semiconductor layer of the present invention is an electron transport, electron injection or charge generation layer; alternatively an electron transport or charge generation layer.

According to one embodiment of the invention, the at least one photoactive layer is a light-emitting layer.

According to one embodiment of the invention the organic electronic device comprises a first and a second light-emitting layer, wherein the organic semiconductor layer is arranged between the first and the second light-emitting layer.

According to one embodiment of the invention the organic electronic device comprises a first, a second and a third light-emitting layer, wherein the organic semiconductor layer is arranged between the first and the second light-emitting layer and/or between the second and third light-emitting layer.

According to one embodiment of the invention, the organic semiconductor layer is a charge generation layer, alternatively an n-type charge generation layer.

According to one embodiment of the invention the electronic organic device is an electroluminescent device, preferably an organic light emitting diode.

The present invention furthermore relates to a display device comprising an organic electronic device according to the present invention.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound.

In case that the redox n-dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

Preferably, the redox potential of the electrically neutral metal complex and/or of the electrically neutral organic

radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or
(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as redox n-dopants may be e.g. strongly reductive complexes of some transition metals in low oxidation state. Particularly strong redox n-dopants may be selected for example from Cr(II), Mo(II) and/or W(II) guanidinate complexes such as $W_2(hpp)_4$, as described in more detail in WO2005/086251.

Electrically neutral organic radicals suitable as redox n-dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form.

It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the n-dopant may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The redox dopant may be essentially non-emissive.

According to one embodiment of the invention the organic semiconductor layer is a charge-generation layer.

According to one embodiment of the invention the organic semiconductor layer is an n-type charge-generation layer.

According to one embodiment of the invention the organic semiconductor layer is a electron-transport layer.

According to one embodiment of the invention, the at least one photoactive layer is a light-emitting layer.

According to one embodiment of the invention the organic electronic device comprises a first and a second light-emitting layer, wherein the organic semiconductor layer is arranged between the first and the second light-emitting layer

According to one embodiment of the invention the electronic organic device is an electroluminescent device, preferably an organic light emitting diode

The present invention furthermore relates to a display device comprising an organic electronic device according to the present invention.

<u>Further layers</u>

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a holetransporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Photoactive layer (PAL)*

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be a light-emitting layer or a light-absorbing layer.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp) 3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the

general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

According to an embodiment, a hole blocking layer is arranged between the at least one photoactive layer and the organic semiconductor layer comprising compound of formula (1).

According to an embodiment, a hole blocking layer is arranged between the at least one photoactive layer and the organic semiconductor layer comprising compound of formula (1), wherein the organic semiconductor layer comprising compound of formula (1) further comprises a redox n-dopant.

According to an embodiment, a hole blocking layer is arranged between the at least one photoactive layer and the organic semiconductor layer comprising compound of formula (1), wherein the organic semiconductor layer comprising compound of formula (1) further comprises a metal or a metal organic complex, alternatively a metal or a lithium organic complex.

According to an embodiment, a hole blocking layer is arranged between the at least one photoactive layer and the organic semiconductor layer comprising compound of formula (1), wherein the organic semiconductor layer comprising compound of formula (1) further comprises a metal.

*Electron transport layer (ETL)*

The OLED according to the present invention may comprise an electron transport layer (ETL). In accordance with one preferred embodiment of the invention, the electron transport layer may be the inventive organic semiconductor layer comprising the inventive compound represented by the general Formula (1) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (1) as defined above as the organic electron transport matrix (ETM) material. The electron transport layer may comprise, besides or instead of the compound represented by the general Formula (1), further ETM materials known in the art. Likewise, the electron transport layer may comprise as the only electron transport matrix material the compound represented by general Formula (1). In case that the inventive organic electronic device comprises more than one electron transport layers, the compound represented by the general Formula (1) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970371 A1 or WO 2013/079217 A1.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, $Li_2O$, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the organic semiconductor layer comprising the compound of Formula (1).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

*Charge generation layer (CGL)*

The charge generation layer (CGL) may comprise a p- type charge generation layer (p-CGL) and an n-type charge generation layer (n-CGL). An interlayer may be arranged between the p-CGL and the -n-CGL.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (1). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline) aluminum, benzazole derivatives, and silole derivatives.

The hole generating layer is arranged in direct contact to the n-type charge generation layer.

According to one aspect of the present invention, the organic semiconductor layer is arranged between the first and second emission layer and further comprises a redox n-dopant.

According to one aspect of the present invention, the organic semiconductor layer is arranged between the first and second emission layer and further comprises a metal.

According to one aspect of the present invention, the organic semiconductor layer is arranged between the first and second emission layer and further comprises a metal selected from alkali, alkaline earth and rare earth metals.

According to one aspect of the present invention, the organic semiconductor layer comprising compound of formula (1) is arranged between the first and second emission layer and a further organic semiconductor layer comprising compound of formula (1) is arranged between the second emission layer and the cathode.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the further organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a redox n-dopant, and the further organic semiconductor layer comprising compound of formula (1) further comprises a redox n-dopant.

According to one aspect of the present invention, the organic semiconductor layer comprising compound of formula (1) is arranged between the first and second emission layer and a further organic semiconductor layer comprising compound of formula (1) is arranged between the second emission layer and the cathode; wherein the organic semiconductor layer comprising compound of formula (1) further comprises a metal, and the further organic semiconductor layer comprising compound of formula (1) further comprises a redox n-dopant.

*Organic light-emitting diode (OLED)*

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the electron transport layer, first electron transport layer, n-type charge generation layer and/or second electron transport layer.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

Organic electronic device

The organic electronic device according to the invention may be a light emitting device, or a photovoltaic cell, and

preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spincoating, printing, casting; and/or
- slot-die coating.

According to various embodiments of the present invention, there is provided a method using:

- a first deposition source to release the compound of Formula (1) according to the invention, and
- a second deposition source to release the metal, a metal salt or an alkali or alkaline earth metal complex; alternatively an organic alkali or alkaline earth metal complex; alternatively 8-hydroxyquinolinolato lithium;

the method comprising the steps of forming the organic semiconductor layer; whereby for an organic light-emitting diode (OLED):

- the organic semiconductor layer is formed by releasing the compound of Formula (1) according to the invention from the first deposition source and a metal, a metal salt or an alkali or alkaline earth metal complex; alternatively an organic alkali or alkaline earth metal complex; alternatively 8-hydroxyquinolinolato lithium, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconductor layer and the cathode electrode.

According to various embodiments of the present invention, the method may further comprise forming an electron injection layer on the organic semiconductor layer. However, according to various embodiments of the OLED of the present invention, the OLED may not comprise an electron injection layer.

According to various embodiments, the OLED may have the following layer structure, wherein the layers having the following order:

anode, hole injection layer, first hole transport layer, second hole transport layer, emission layer, optional hole blocking layer, organic semiconductor layer comprising a compound of Formula (1) according to the invention, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconductor layer comprising a compound according to Formula (1) may further comprise a layer comprising a radialene compound and/or a quinodimethane compound.

In one embodiment, the radialene compound and/or the quinodimethane compound may be substituted with one

or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

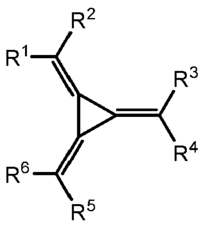
(XX)
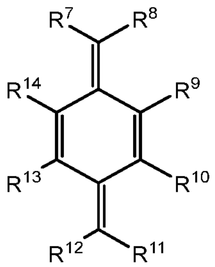
(XXIa)
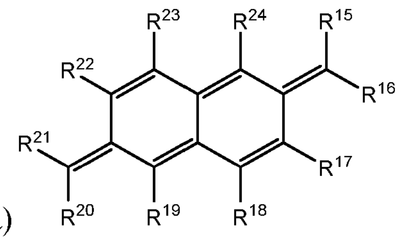
(XXIb),

wherein (as an exception different to the description above) $R^1$, $R^2$, $R^3$, $R^4$, $R^5$, $R^6$, $R^7$, $R^8$ $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

According to one embodiment of the present invention, the organic semiconductor layer comprising compound of formula (1) is adjacent to a layer comprising a compound of formula (XX), (XXIa) or (XXIb).

According to one embodiment of the present invention, the organic semiconductor layer comprising compound of formula (1) is in direct contact to a layer comprising a compound of formula (XX), (XXIa) or (XXIb).

Hereinafter, the embodiments are illustrated in more detail with reference to examples. However, the present disclosure is not limited to the following examples. Reference will now be made in detail to the exemplary aspects.

## Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode 120, a photoactive layer (PAL) 125, an organic semiconductor layer comprising a compound of formula (1) 160. The organic semiconductor layer comprising compound of formula (1) 160 is formed on the PAL 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconductor layer comprising a compound of Formula (1) may be an ETL.

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconductor layer comprising a compound of Formula (1) may be an n-type CGL.

Preferably, the organic semiconductor layer comprising a compound of Formula (1) may be the first ETL, n-type CGL and/or second ETL.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

## Detailed description

The invention is furthermore illustrated by the following examples which are illustrative only and non-binding.

## Experimental part

### 1) General procedure for the synthesis of compound of Formula 1

In a first step intermediate compound 1 was synthesized as follow:

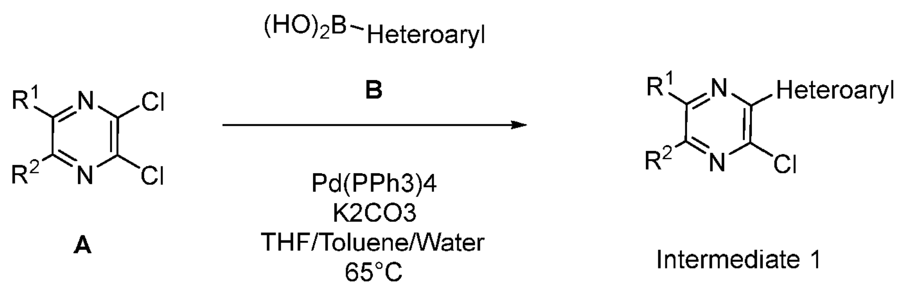

A flask was flushed with nitrogen and charged with reagent **A** (33.2 mmol), reagent **B** (26.6 mmol), $Pd(PPh_3)_4$ (1.7 mmol), and $K_2CO_3$ (83.0 mmol). A mixture of deaerated Toluene/THF/water (1:1:1 81 mL) was added and the reaction mixture was heated to 65 °C under a nitrogen atmosphere during two hours. After cooling down to 0°C, the resulting precipitate was isolated by suction filtration washed with toluene (3 x 20 mL) and dried. The crude product was then dissolved in chloroform (300 mL) and the organic phase was washed with water (3 x 100 mL). After drying over $MgSO_4$, the organic phase was filtered through a silicagel pad. After rinsing with additional chloroform (1000 mL), the solvents were removed under reduced pressure. Dichloromethane (20 mL) and isopropanol (40 mL) were added and then the dichloromethane was slowly removed under reduced pressure to induce precipitation. After filtration, the crude solid was dissolved in dichloromethane (30 mL) and hexane (60 mL) was added to induce precipitation. The precipitate was collected by suction filtration to yield intermediate 1 after drying. Purity was determined by HPLC.

In the second step compound of formula 1 is synthesized using standard borylation reaction as follow:

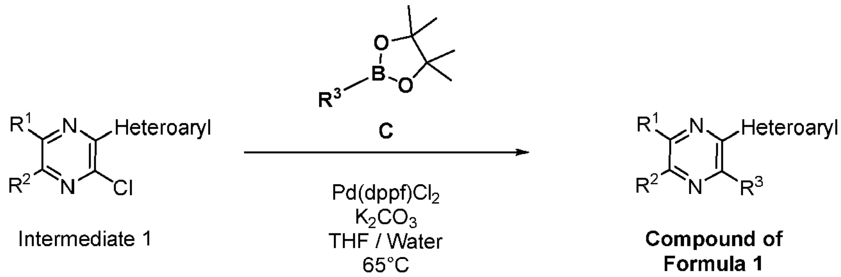

Intermediate 1

**Compound of Formula 1**

A flask was flushed with nitrogen and charged with intermediate 1 (11.9 mmol), reagent C (11.9 mmol), Pd(dppf) $Cl_2$ (0.2 g, 0.24 mmol), and $K_2CO_3$ (3.3 g, 23.8 mmol). A mixture of deaerated THF/water (4:1, 60 mL) was added and the reaction mixture was heated to 65 °C under a nitrogen atmosphere overnight. After cooling down to room temperature, the resulting precipitate was isolated by suction filtration, washed with hexane (250 mL), water (1000 mL) and methanol (2x 50 mL). The crude product was dried, dissolved in hot chlorobenzene (500 mL) and filtered through a silicagel pad. After rinsing with additional chlorobenzene (500 mL), the solvents were partially removed under reduced pressure. Hexane (200 mL) was added to induce precipitation. After filtration, the product was recrystallized in chlorobenzene (100 mL) to yield compound of formula 1 after drying. Final purification was achieved by sublimation.

**2) General procedure for the synthesis of compound of Formula 1**

In a first step intermediate compound 1 was synthesized as follow:

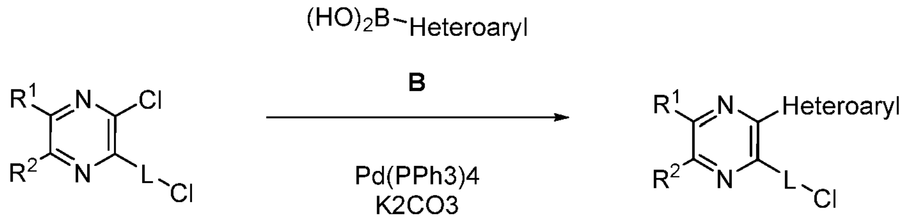

**A-1**

Intermediate 1-1

A flask was flushed with nitrogen and charged with Reagent **A-1** (59.9 mmol), reagent **B** (75.0 mmol), $Pd(PPh_3)_2Cl_2$ (2.1 g, 3.0 mmol), and $K_2CO_3$ (33.1 g, 239.6 mmol). A mixture of deaerated THF/water (3:1, 400 mL) was added and the reaction mixture was heated to 55 °C under a nitrogen atmosphere during three hours. After cooling down to room temperature, reaction mixture was washed with water and the organic phase was dried over $MgSO_4$. Solvents were removed under reduced pressure, residue was dissolved in hot dichloromethane, embedded onto silicagel and purified by column chromatography using as eluent dichloromethane / petroleum ether (3:10, 1 L - 3.5:10, 1 L - 4:10, 2 L) and dichloromethane. Solvents were evaporated and solid was dissolved in hot dichloromethane, giving a turbid solution. Remaining solids were filtered off and discarded. Solution was cooled down, solvents were partially removed under reduced pressure and petroleum ether (900 mL) was added to induce precipitation. Solid was filtered and recrystallized in dioxane (270 mL) to yield intermediate 1-1.

In the second step compound of formula 1-1 is synthesized using standard borylation reaction as follow:

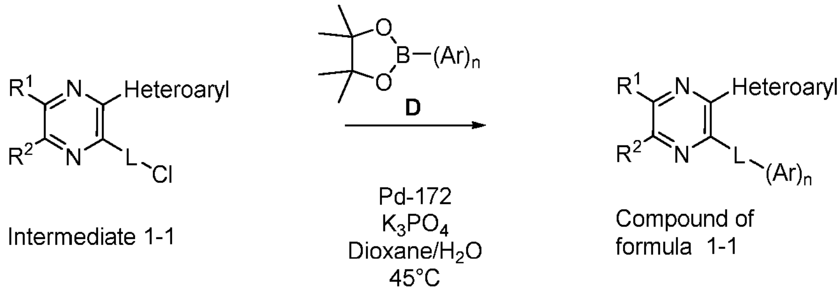


Intermediate 1-1

Compound of formula 1-1

A flask was flushed with nitrogen and charged Intermediate 1-1 (59.9 mmol), reagent **D** (12.8 g, 75.0 mmol), $Pd(PPh_3)_2Cl_2$ (2.1 g, 3.0 mmol), and $K_2CO_3$ (33.1 g, 239.6 mmol). A mixture of deaerated THF/water (3:1, 400 mL) was added and the reaction mixture was heated to 55 °C under a nitrogen atmosphere during three hours. After cooling down to room temperature, reaction mixture was washed with water and the organic phase was dried over $MgSO_4$. Solvents were removed under reduced pressure, residue was dissolved in hot dichloromethane, embedded onto silicagel and purified by column chromatography using as eluent dichloromethane / petroleum ether (3:10, 1 L - 3.5:10, 1 L - 4:10, 2 L) and dichloromethane. Solvents were evaporated and solid was dissolved in hot dichloromethane, giving a turbid solution. Remaining solids were filtered off and discarded. Solution was cooled down, solvents were partially removed under reduced pressure and petroleum ether (900 mL) was added to induce precipitation. Solid was filtered and recrystallized in dioxane (270 mL) to yield compound of formula 1-1.

**Synthesis of 2,3,5-triphenyl-6-(4-(4,4,5,5-tetramethyl-1,3,2-dioxaborolan-2-yl)phenyl)pyrazine**

First step: Synthesis of 2-chloro-3-(dibenzo[b,d]furan-3-yl)-5,6-diphenylpyrazine

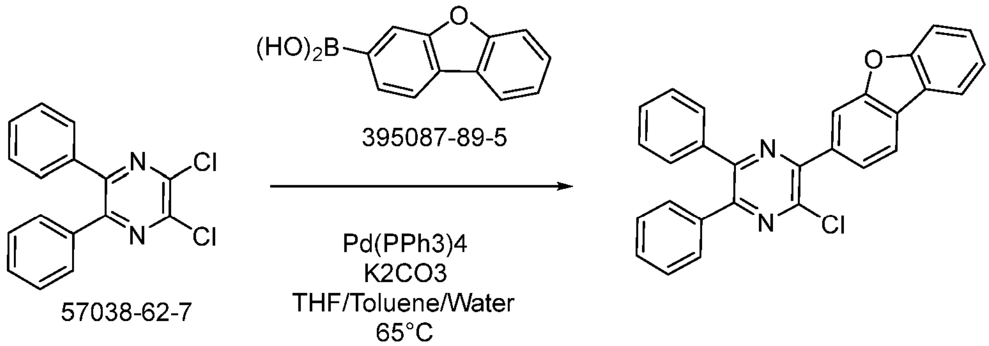


395087-89-5

57038-62-7

A flask was flushed with nitrogen and charged with 2,3-dichloro-5,6-diphenylpyrazine (10.0 g, 33.2 mmol), dibenzo[b,d]furan-3-ylboronic acid (5.6 g, 26.6 mmol), $Pd(PPh_3)_4$ (1.9 g, 1.7 mmol), and $K_2CO_3$ (11.5 g, 83.0 mmol). A mixture of deaerated Toluene/THF/water (1:1:1, 81 mL) was added and the reaction mixture was heated to 65 °C under a nitrogen atmosphere during two hours. After cooling down to 0°C, the resulting precipitate was isolated by suction filtration washed with toluene (3 x 20 mL) and dried. The crude product was then dissolved in chloroform (300 mL) and the organic phase was washed with water (3 x 100 mL). After drying over $MgSO_4$, the organic phase was filtered through a silicagel pad. After rinsing with additional chloroform (1000 mL), the solvents were removed under reduced pressure. Dichloromethane (20 mL) and isopropanol (40 mL) were added and then the dichloromethane was slowly removed under reduced pressure to induce precipitation. After filtration, the crude solid was dissolved in dichloromethane (30 mL) and hexane (60 mL) was added to induce precipitation. The precipitate was collected by suction filtration to yield 3.4 g (30%) of 2-chloro-3-(dibenzo[b,d]furan-3-yl)-5,6-diphenylpyrazine after drying. HPLC: 98.8%

Second step: 2,3,5-triphenyl-6-(4-(4,4,5,5-tetramethyl-1,3,2-dioxaborolan-2-yl)phenyl)pyrazine was synthesized from 943442-81-7, using standard borylation reaction

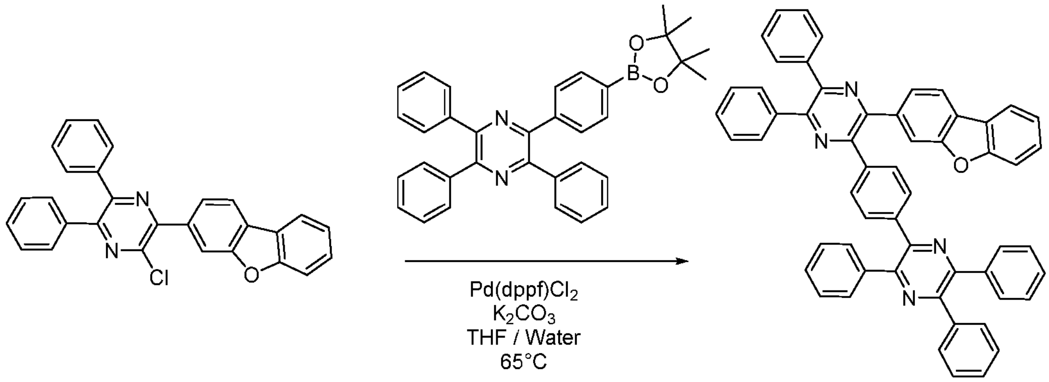


A flask was flushed with nitrogen and charged with 2-chloro-3-(dibenzo[b,d]furan-3-yl)-5,6-diphenylpyrazine (5.1 g, 11.9 mmol), 2,3,5-triphenyl-6-(4-(4,4,5,5-tetramethyl-1,3,2-dioxaborolan-2-yl)phenyl)pyrazine (6.1 g, 11.9 mmol), Pd(dppf)$Cl_2$ (0.2 g, 0.24 mmol), and $K_2CO_3$ (3.3 g, 23.8 mmol). A mixture of deaerated THF/water (4:1, 60 mL) was added and the reaction mixture was heated to 65 °C under a nitrogen atmosphere overnight. After cooling down to room temperature, the resulting precipitate was isolated by suction filtration, washed with hexane (250 mL), water (1000 mL) and methanol (2x 50 mL). The crude product was dried, dissolved in hot chlorobenzene (500 mL) and filtered through a silicagel pad. After rinsing with additional chlorobenzene (500 mL), the solvents were partially removed under reduced pressure. Hexane (200 mL) was added to induce precipitation. After filtration, the product was recrystallized in chlorobenzene (100 mL) to yield 5.7 g (61%) of 2,3,5-triphenyl-6-(4-(4,4,5,5-tetramethyl-1,3,2-dioxaborolan-2-yl)phenyl)pyrazine after drying. Final purification was achieved by sublimation. HPLC/ESI-MS: 99.8%, m/z = 781 ([M+H]$^+$), 781 ([M+H]$^+$).

**Synthesis of 2-(dibenzo[b,d]furan-3-yl)-5,6-diphenyl-3-(4-(pyridin-3-yl)phenyl)pyrazine**

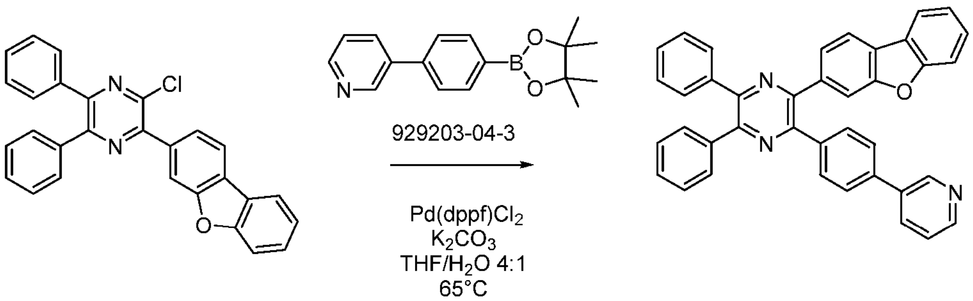


A flask was flushed with nitrogen and charged with 2-chloro-3-(dibenzo[b,d]furan-3-yl)-5,6-diphenylpyrazine (6.0 g, 13.7 mmol), 3-(4-(4,4,5,5-tetramethyl-1,3,2-dioxaborolan-2-yl)phenyl)pyridine (4.2 g, 15.1 mmol), Pd(dppf)$Cl_2$ (0.2 g, 0.27 mmol), and $K_2CO_3$ (3.8 g, 27.4 mmol). A mixture of deaerated THF/water (4:1, 70 mL) was added and the reaction mixture was heated to 65 °C under a nitrogen atmosphere overnight. After cooling down to room temperature, the resulting precipitate was isolated by suction filtration washed with water (2 x 50 mL) and with methanol (2x 50 mL). The crude product was dried, dissolved in dichloromethane (150 mL) and washed with an aqueous solution of sodium diethyldithiocarbamate trihydrate and then with water. After drying over $MgSO_4$, it was filtered through a silicagel pad. After rinsing with additional dichloromethane (1000 mL) and dichloromethane/methanol (100:3, 2500 mL), solvents were partially removed under reduced pressure to 100 mL. Suspension was filtered to yield 4.8 g (63%) of 2-(dibenzo[b,d]furan-3-yl)-5,6-diphenyl-3-(4-(pyridin-3-yl)phenyl)pyrazine after drying. Final purification was achieved by sublimation. HPLC/ESI-MS: 99.9%, m/z = 552 ([M+H]$^+$).

**Synthesis of 4''-(3-(dibenzo[b,d]furan-3-yl)-5,6-diphenylpyrazin-2-yl)-[1,1':4',1''-terphenyl]-4-carbonitrile**

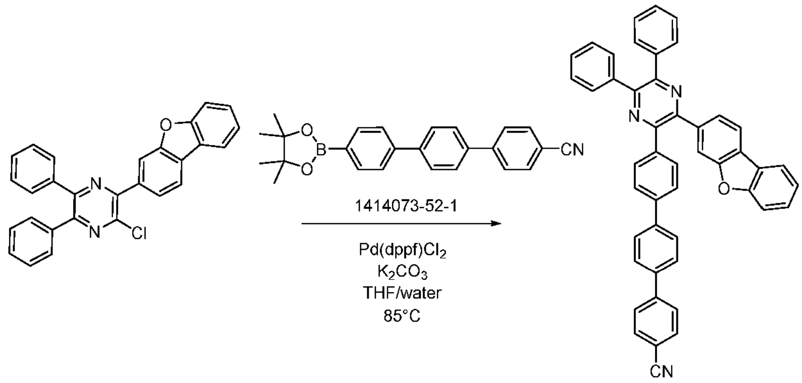


A flask was flushed with nitrogen and charged with 2-chloro-3-(dibenzo[b,d]furan-3-yl)-5,6-diphenylpyrazine (2.8 g, 6.4 mmol), 4"-(4,4,5,5-tetramethyl-1,3,2-dioxaborolan-2-yl)-[1,1':4',1"-terphenyl]-4-carbonitrile (2.6 g, 6.8 mmol), Pd(dppf)$Cl_2$ (0.09 g, 0.13 mmol), and $K_2CO_3$ (1.8 g, 12.9 mmol). A mixture of deaerated THF/water (10:1, 55 mL) was added and the reaction mixture was heated to 85 °C under a nitrogen atmosphere over two days. After cooling down to room temperature, solvents were removed under reduced pressure, the residue was dissolved in dichloromethane (100 mL), washed with water (3 x 100 mL) and dried over $MgSO_4$. Residue was embedded in silicagel and filtered through a silicagel pad, using as eluent hexane / dichloromethane (3:2, 500 mL) and hexane / dichloromethane (1:1, 1 L). Suspension was filtered to yield 2.5 g (60%) of 4"-(3-(dibenzo[b,d]furan-3-yl)-5,6-diphenylpyrazin-2-yl)-[1,1':4',1"-terphenyl]-4-carbonitrile after drying. Final purification was achieved by sublimation. HPLC/ESI-MS: 99.9%, m/z = 652 ($[M+H]^+$).

**Synthesis of (4'-(3-(dibenzo[b,d]furan-3-yl)-5,6-diphenylpyrazin-2-yl)-[1,1'-biphenyl]-3-yl)dimethylphosphine oxide**

First step: Synthesis of 2-(4-chlorophenyl)-3-(dibenzo[b,d]furan-3-yl)-5,6-diphenylpyrazine

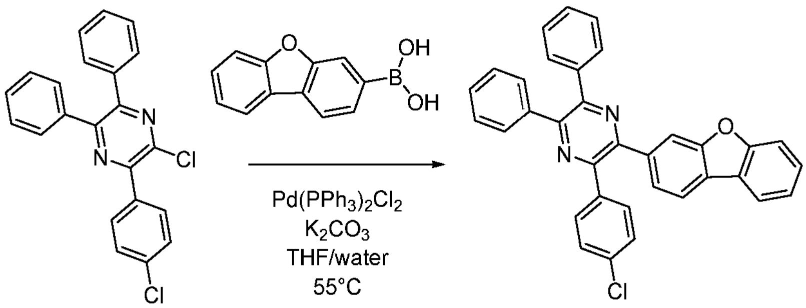


A flask was flushed with nitrogen and charged with 2-chloro-3-(4-chlorophenyl)-5,6-diphenylpyrazine (22.8 g, 59.9 mmol), dibenzo[b,d]furan-3-ylboronic acid (12.8 g, 75.0 mmol), $Pd(PPh_3)_2Cl_2$ (2.1 g, 3.0 mmol), and $K_2CO_3$ (33.1 g, 239.6 mmol). A mixture of deaerated THF/water (3:1, 400 mL) was added and the reaction mixture was heated to 55 °C under a nitrogen atmosphere during three hours. After cooling down to room temperature, reaction mixture was washed with water and the organic phase was dried over $MgSO_4$. Solvents were removed under reduced pressure, residue was dissolved in hot dichloromethane, embedded onto silicagel and purified by column chromatography using as eluent dichloromethane / petroleum ether (3:10, 1 L - 3.5:10, 1 L - 4:10, 2 L) and dichloromethane. Solvents were evaporated and solid was dissolved in hot dichloromethane, giving a turbid solution. Remaining solids were filtered off and discarded. Solution was cooled down, solvents were partially removed under reduced pressure and petroleum ether (900 mL) was added to induce precipitation. Solid was filtered and recrystallized in dioxane (270 mL) to yield 55.9 g (96%) of 2-(4-chlorophenyl)-3-(dibenzo[b,d]furan-3-yl)-5,6-diphenylpyrazine after drying. HPLC: 99.0%

Second step: (4'-(3-(dibenzo[b,d]furan-3-yl)-5,6-diphenylpyrazin-2-yl)-[1,1'-biphenyl]-3-yl)dimethylphosphine oxide was synthesized using standard borylation reaction

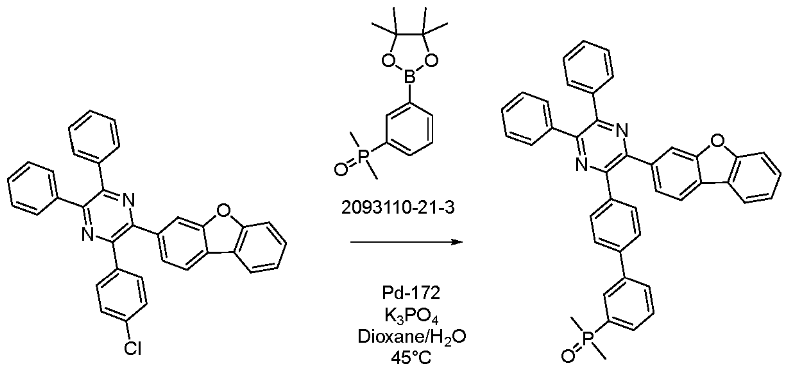


A flask was flushed with nitrogen and charged with 2-(4-chlorophenyl)-3-(dibenzo[b,d]furan-3-yl)-5,6-diphenylpyrazine (10.0 g, 19.6 mmol), dimethyl(3-(4,4,5,5-tetramethyl-1,3,2-dioxaborolan-2-yl)phenyl)phosphine oxide (7.1 g, 25.6 mmol), chloro(crotyl)(2-dicyclohexylphosphino-2',6'-dimethoxybiphenyl)palladium(II) (0.24 g, 0.4 mmol), and $K_3PO_4$ (8.3 g, 39.3 mmol). A mixture of deaerated Dioxane/water (4:1) was added and the reaction mixture was heated to 45 °C under a nitrogen atmosphere during three days. After cooling down to room temperature, the resulting precipitate was isolated by suction filtration washed with dioxane (3 x 20 mL), with water (800 mL) and with methanol (2x 50 mL). The crude product was dried, dissolved in dichloromethane/methanol (100:2, 400 mL) and filtered through a silicagel pad. After rinsing with additional dichloromethane/methanol (100:2, 1700 mL + 100:5, 400 mL), the solvents were partially removed under reduced pressure. Hexane was added to induce precipitation. After filtration, the product was recrystallized in toluene to yield 7.5 g (61%) of (4'-(3-(dibenzo[b,d]furan-3-yl)-5,6-diphenylpyrazin-2-yl)-[1,1'-biphenyl]-3-yl)dimethylphosphine oxide after drying. Final purification was achieved by sublimation. HPLC/ESI-MS: 100%, m/z = 627 ([M+H]$^+$).

Table 1 Properties of compound of Formula 1

| Compounds of Formula 1 | HOMO (eV) | LUMO (eV) | Diplole moment (Debye) | mp (°C) | Tg (°C) | TRO (°C) |
|---|---|---|---|---|---|---|
| 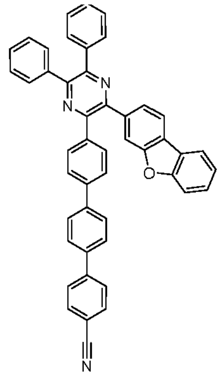 I-1 | -5.70 | -1.90 | 6.19 | 277 | 140 | 258 |
| 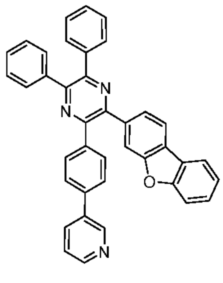 I-2 | -5.56 | -1.80 | 2.13 | 281 | 113 | 367 |

(continued)

| Compounds of Formula 1 | HOMO (eV) | LUMO (eV) | Diplole moment (Debye) | mp (°C) | Tg (°C) | TRO (°C) |
|---|---|---|---|---|---|---|
| 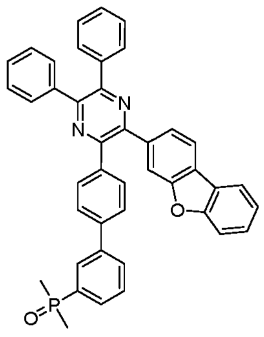 I-3 | -5.72 | -1.74 | 3.57 | -- | 131 | 254 |
| 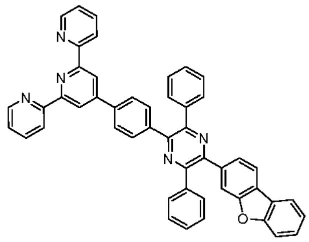 I-4 | -5.56 | -1.86 | 2.54 | 333 | 151 | 284 |
| 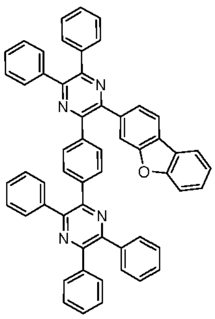 I-5 | -5.46 | -1.82 | 0.71 | 311 | 151 | 284 |

General procedure 1 for fabrication of OLEDs

A top emission device was made by depositing an anode of 100 nm thick Ag on a glass substrate.

Then, 92 vol.-% N-([1,1'-biphenyl]-4-yl)-9,9-dimethyl-N-(4-(9-phenyl-9H-carbazol-3-yl)phenyl)-9H-fluoren-2-amine (CAS 1242056-42-3) with 8 vol.-% 2,2',2"-(cyclopropane-1,2,3-triylidene)tris(2-(p-cyanotetrafluorophenyl)acetonitrile) was vacuum deposited on the anode, to form a HIL having a thickness of 10 nm. Then, N-([1,1'-biphenyl]-4-yl)-9,9-dimethyl-N-(4-(9-phenyl-9H-carbazol-3-yl)phenyl)-9H-fluoren-2-amine was vacuum deposited on the HIL, to form a HTL having a thickness of 128 nm.

Then, N-(4-(dibenzo[b,d]furan-4-yl)phenyl)-N-(4-(9-phenyl-9H-fluoren-9-yl)phenyl)-[1,1'-biphenyl]-4-aminewas vacuum deposited on the HTL, to form an electron blocking layer (EBL) having a thickness of 5 nm.

Then, 97 vol.-% H09 (Sun Fine Chemicals, Korea) as EML host and 3 vol.-% BD200 (Sun Fine Chemicals, Korea) as fluorescent blue dopant were deposited on the EBL, to form a blue-emitting EML with a thickness of 20 nm.

Then the auxiliary electron transport layer (ETL-1) was formed with a thickness of 5 nm by depositing 2 -(3'-(9,9-dimethyl-9H-fluoren-2-yl)-[1,1'-biphenyl]-3-yl)-4,6-diphenyl-1,3,5-triazineon the emission layer (EML).

Then, the electron transporting layer 2 (ETL-2) was formed on the auxiliary electron transport layer (ETL-1) by depositing the compound of Formula (1) according to the inventive example 1 to example X and comparative compound 1 according to the comparative example 1 with a the thickness of 31 nm. The electron transport layer 2 (ETL-2) comprises 50 wt.-% matrix compound and 50 wt.-% of LiQ, see Table 2.

Then, the electron injection layer was formed on the electron transporting layer 2 by deposing Yb with a thickness of 2 nm.

Ag:Mg (90: 10) was co-deposited at a rate of 0.01 to 1 Å/s at 10-7 mbar to form a cathode with a thickness of 11 nm.

The OLED stack is protected from ambient conditions by encapsulation of the device with a glass slide. Thereby, a cavity is formed, which includes a getter material for further protection.

General procedure for fabrication of a tandem OLED device.

A top emission device was made by depositing an anode of 100 nm thick Ag on a glass substrate.

Then, 97 vol.-% Biphenyl-4-yl(9,9-diphenyl-9H-fluoren-2-yl)-[4-(9-phenyl-9H-carbazol-3-yl)phenyl]-amine (CAS 1242056-42-3) with 3 vol.-% 2,2',2"-(cyclopropane-1,2,3-triylidene)tris(2-(p-cyanotetrafluorophenyl)acetonitrile) was vacuum deposited on the anode, to form a HIL having a thickness of 10 nm.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a first HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form a first electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a first blue-emitting EML with a thickness of 20 nm.

Then the first hole blocking layer is formed with a thickness of 5 nm by 4'-(4-(4-(4,6-diphenyl-1,3,5-triazin-2-yl) phenyl)naphthalen-1-yl)-[1,1'-biphenyl]-4-carbonitrile on the emission layer.

Then, the first electron transporting layer having a thickness of 25 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile. The electron transport layer (ETL) comprises 50 wt.-% matrix compound and 50 wt.-% of LiQ.

Then the first n-CGL was formed on ETL with a thickness of 15 nm. The n-CGL comprises organic compound of formula 1 and metal dopant (Table 3). Then the p-CGL was formed on n-CGL with a thickness of 10 nm on n-CGL by depositing 90 vol.-% Biphenyl-4-yl(9,9-diphenyl-9H-fluoren-2-yl)-[4-(9-phenyl-9H-carbazol-3-yl)phenyl]-amine (CAS 1242056-42-3) with10 vol.-% 2,2',2"-(cyclopropane-1,2,3-triylidene)tris(2-(p-cyanotetrafluorophenyl)acetonitrile).

Then, Biphenyl-4-yl(9,9-diphenyl-9H-fluoren-2-yl)-[4-(9-phenyl-9H-carbazol-3-yl) phenyl]-amine was vacuum deposited to form a second HTL having a thickness of 10 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form a second electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a second blue-emitting EML with a thickness of 20 nm.

Then the second hole blocking layer is formed with a thickness of 5 nm by 4'-(4-(4-(4,6-diphenyl-1,3,5-triazin-2-yl)phenyl)naphthalen-1-yl)-[1,1'-biphenyl]-4-carbonitrile on the emission layer.

Then, the second electron transporting layer having a thickness of 25 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile. The electron transport layer (ETL) comprises 50 wt.-% matrix compound and 50 wt.-% of LiQ.

Then the second n-CGL was formed on ETL with a thickness of 15 nm. The n-CGL comprises organic compound of formula 1 and metal dopant (Table 3). Then the p-CGL was formed on n-CGL with a thickness of 10 nm on n-CGL by depositing 90 vol.-% Biphenyl-4-yl(9,9-diphenyl-9H-fluoren-2-yl)-[4-(9-phenyl-9H-carbazol-3-yl)phenyl]-amine (CAS 1242056-42-3) with10 vol.-% 2,2',2"-(cyclopropane-1,2,3-triylidene)tris(2-(p-cyanotetrafluorophenyl)acetonitrile).

Then, Biphenyl-4-yl(9,9-diphenyl-9H-fluoren-2-yl)-[4-(9-phenyl-9H-carbazol-3-yl) phenyl]-amine was vacuum deposited on the HIL, to form a third HTL having a thickness of 10 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form third electron blocking layer (EBL) having a thickness of 5 nm.

Then 97 vol.-% H09 (Sun Fine Chemicals, South Korea) as EML host and 3 vol.-% BD200 (Sun Fine Chemicals, South Korea) as fluorescent blue dopant were deposited on the EBL, to form a third blue-emitting EML with a thickness of 20 nm.

Then the third hole blocking layer is formed with a thickness of 5 nm by 4'-(4-(4-(4,6-diphenyl-1,3,5-triazin-2-yl) phenyl)naphthalen-1-yl)-[1,1'-biphenyl]-4-carbonitrile on the emission layer.

Then, the third electron transporting layer having a thickness of 25 nm is formed on the hole blocking layer by depositing 4'-(4-(4-(4,6-diphenyl-1,3,5-triazin-2-yl)phenyl)naphthalen-1-yl)-[1,1'-biphenyl]-4-carbonitrile. The electron transport layer (ETL) comprises 50 wt.-% matrix compound and 50 wt.-% of LiQ.

An electron injecting layer is formed by depositing Yb with a thinkness of 2nm.

A cathode with a thickness of 100 nm is formed by depositing Ag:Mg (90:10) at the rate of 0.01 to 1 Å/s at $10^{-7}$ mbar.

A caping layer is formed by depositing N,N-bis(4-(dibenzo[b,d]furan-4-yl)phenyl)-[1,1':4',1 "-terphenyl]-4-amine

with a thickness of 60 nm.

The OLED stack is protected from ambient conditions by encapsulation of the device with a glass slide. Thereby, a cavity is formed, which includes a getter material for further protection.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m$^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

Lifetime LT of the device is measured at ambient conditions (20°C) and 30 mA/cm$^2$, using a Keithley 2400 sourcemeter, and recorded in hours.

The brightness of the device is measured using a calibrated photo diode. The lifetime LT is defined as the time till the brightness of the device is reduced to 97 % of its initial value.

***Technical Effect***

Surprisingly, it was found that the organic electronic device comprising organic semiconductor layer comprising compound of formula 1 according to the present invention solve the problem underlying by being superior over the organic electronic device known in the art, in particular with respect to operating voltage, which is important for reducing power consumption and increasing battery life, for example of a mobile display device. At the same time the cd/A efficiency, also referred to as current efficiency is kept at a similar or even improved level. Long life time at high current density is important for the longevity of a device which run at high brightness.

The beneficial effect of the invention on the performance of organic electronic devices can be seen in Table 2 and Table 3.

Table 2 shows the performance of OLED device comprising an ETL comprising compound of formula 1.

| Example | Matrix compound | Alkali organic complex | Conc. of matrix compound and alkali organic complex (vol.-%) | Thickness ETL (nm) | Operating voltage at 10 mA/cm$^2$ (V) | cd/A efficiency at 10 mA/cm$^2$ (cd/A) |
|---|---|---|---|---|---|---|
| Comparative example 1 | 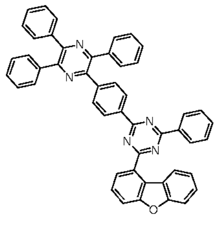 C-1 | LiQ | C-1:LiQ (50:50) | 31 | 3.9 | 6.1 |
| Comparative example 2 | 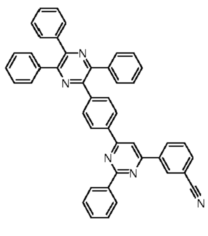 C-2 | LiQ | C-2:LiQ (50:50) | 31 | 4.0 | 6.2 |

(continued)

| Example | Matrix compound | Alkali organic complex | Conc. of matrix compound and alkali organic complex (vol.-%) | Thickness ETL (nm) | Operating voltage at 10 mA/cm$^2$ (V) | cd/A efficiency at 10 mA/cm$^2$ (cd/A) |
|---|---|---|---|---|---|---|
| Comparative example 3 | 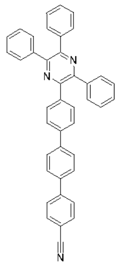 C-3 | LiQ | C-3:LiQ (50:50) | 31 | 3.8 | -- |
| Example 1 | 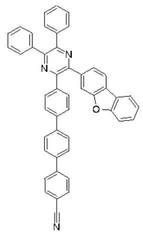 I-1 | LiQ | I-1:LiQ (50:50) | 31 | 3.7 | 7.1 |
| Example 2 | 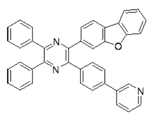 I-2 | LiQ | I-2:LiQ (50:50) | 31 | 3.5 | 7.2 |
| Example 3 | 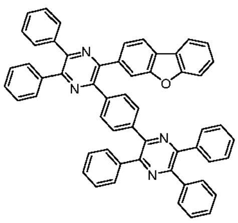 1-5 | LiQ | I-3:LiQ (50:50) | 31 | 3.5 | 7.5 |

The OLED device of inventive Example 1-3 comprising electron transport layer comprising the compound of formula 1 showed an improved efficiency (cd/A at 10 mA/cm$^2$) and low operating voltage (10 mA/cm$^2$ (V)) as compared to the OLED device of comparative example 1-3 comprising electron transport layer comprising comparative compound C-1 to C-3.

Table 3: Performance of a tandem organic electronic device comprising an n-CGL comprising first compound of formula 1 and a metal dopant

| Example | Matrix compound | Metal dopant | 1st and 2nd n-CGL (vol.-%) | Thickness n-CGL (nm) | Operating voltage at 15 mA/cm2 (V) | CEff/(cd/A) [15 mA/cm2] | LT97 30mA/cm2 (h) |
|---|---|---|---|---|---|---|---|
| Comparative Example 4 | 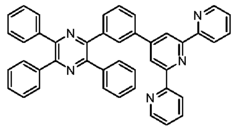 C-4 | Yb | C-4:Yb (99:1) | 15 | 13.5 | 22.4 | 67 |
| Example 4 | 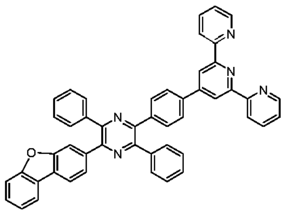 I-4 | Yb | I-4:Yb (99:1) | 15 | 11.2 | 24.1 | 78 |
| Example 5 | 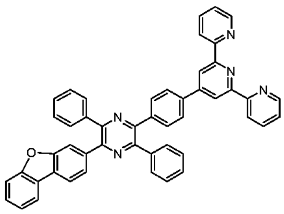 I-4 | Li | I-4:Yb (99:1) | 15 | 10.92 | 24.0 | 77 |

The tandem OLED device of inventive Example 4-5 comprising an n-CGL comprising the compound of formula 1 showed an improved efficiency (cd/A at 10 mA/cm$^2$), long life (LT9730mA/cm$^2$) and low operating voltage (15 mA/cm$^2$ (V)) as compared to the OLED device of Comparative example 4 comprising an n-CGL comprising comparative C-4.

The results show that in comparison with state-of-art reference, the compounds according to invention show a clearly enhanced performance, especially with reference to the efficiency (cd/A at 10 mA/cm$^2$), long lifetime (LT9730mA/cm$^2$) and low operating voltage (10 mA/cm$^2$ (V)). The OLED device of inventive Example 1-3 comprising electron transport layer comprising the compound of formula 1 showed an improved efficiency (cd/A at 10 mA/cm$^2$) and low operating voltage (10 mA/cm$^2$ (V)) as compared to the OLED device of comparative example 1-3 comprising electron transport layer comprising comparative compound C-1 to C-3. The tandem OLED device of inventive Example 4-5 comprising an n-CGL comprising the compound of formula 1 showed an improved efficiency (cd/A at 10 mA/cm$^2$), long lifetime (LT9730mA/cm$^2$) and low operating voltage (15 mA/cm$^2$ (V)) as compared to the OLED device of Comparative example 4 comprising an n-CGL comprising comparative C-4.

Methods:

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Ångstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

## Claims

**1.** An organic electronic device (100) comprising an anode (120), a cathode (190), at least one photoactive layer (125) and at least one organic semiconductor layer (160), wherein the at least one organic semiconductor layer (160) is arranged between the at least one photoactive layer (125) and the cathode (190); and wherein the at least one organic semiconductor layer (160) comprises a $C_s$-symmetric compound of Formula (1):

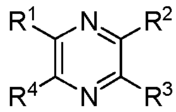

(1)

wherein

$R^1$-$R^4$ are independently selected from a substituted or unsubstituted $C_6$-$C_{36}$ aryl, and a substituted or unsubstituted $C_3$-$C_{36}$ heteroaryl;
wherein at least one of the $R^1$-$R^4$ is selected from a $C_{3-}C_{36}$ heteroaryl group or heteroarylene group which is directly attached to the pyrazine ring in formula 1 and at least three of $R^1$-$R^4$ are different to each other;
wherein, if substituent(s) are present in $R^1$, $R^2$, $R^3$, and $R^4$, the substituent(s) are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, (C=O)$N(R)_2$, $Si(R)_3$, (S=O)R, and

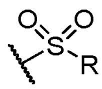,

whereby
Y is O or S; and
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;
wherein two of the $R^1$-$R^4$ are phenyl or naphthenyl;
and wherein the following compounds 1-4 are excluded:

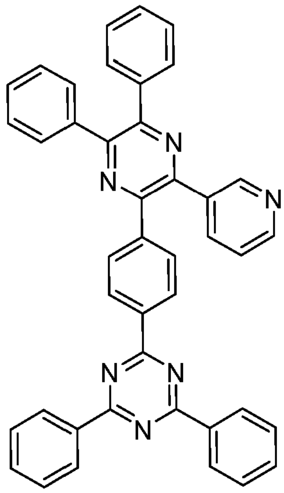

**1** ,

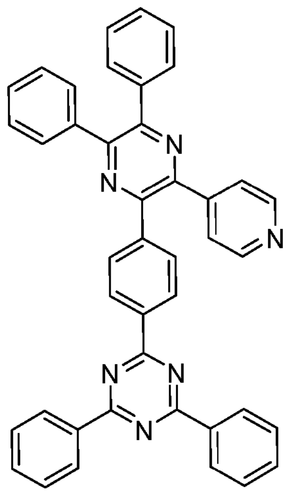

2 ,

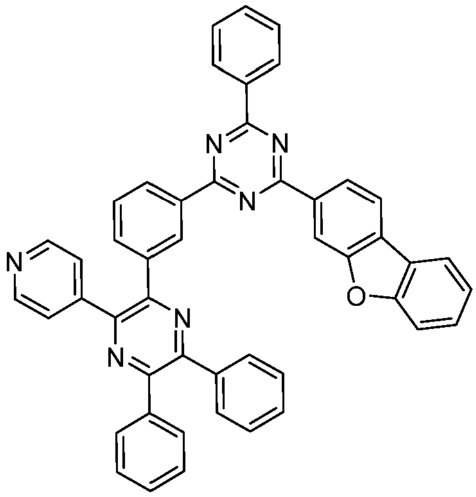

**3** ,

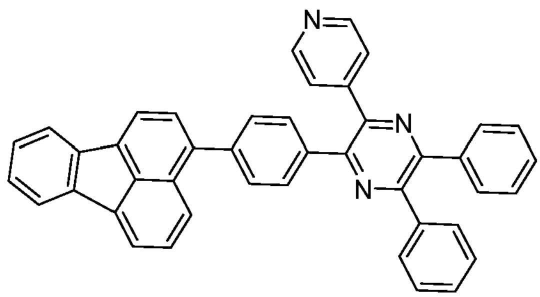

**4** .

2. The organic electronic device (100) of claim 1, wherein the $R^1$ to $R^4$ that are not a substituted or unsubstituted $C_3$-$C_{36}$ heteroaryl or heteroarylene, which is directly attached to the pyrazine ring in formula (1) through a single bond are selected from substituted or unsubstituted $C_6$-$C_{20}$ aryl, and a substituted or unsubstituted $C_3$-$C_{18}$ heteroaryl.

3. The organic electronic device (100) of claim 1 or 2 whereby the at least one of the $R^1$ to $R^4$ that is $C_{3-}C_{36}$ heteroaryl group or heteroarylene group which is directly attached to the pyrazine ring in formula (1) through a single bond is a substituted or unsubstituted $C_3$-$C_{18}$ heteroaryl or heteroarylene.

4. The organic electronic device (100) of any of the claims 1 to 3 whereby the compound has a dipole moment of $\geq$ 0.4 D.

5. The organic electronic device (100) of any of the claims 1 to 4, whereby two of the $R^1$-$R^4$ are phenyl or naphthenyl and in ortho position to each other.

6. The organic electronic device (100) of any of the claims 1 to 5, at least one of the $R^1$ to $R^4$ is selected from formula (2):

$$-L-(Ar)_n \qquad (2)$$

whereby

L is substituted or unsubstituted $C_6$-$C_{36}$ arylene
Ar is selected from a substituted or unsubstituted $C_6$-$C_{36}$ aryl, and a substituted or unsubstituted $C_3$-$C_{36}$ heteroaryl;
n is 1 to 5;
two or more groups Ar may be identical to or different from each other.

7. The organic electronic device (100) of any of the claims 1 to 6, whereby L is chosen from one of the following moieties

A1 to A16:

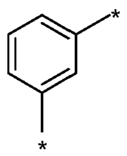
A1

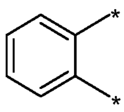
A2

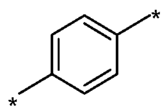
A3

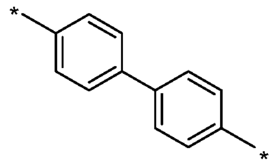
A4

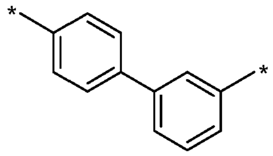
A5

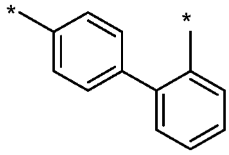
A6

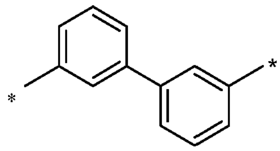
A7

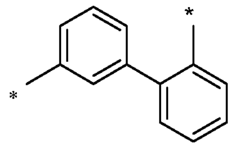
A8

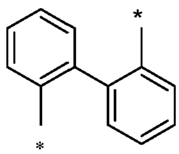
A9

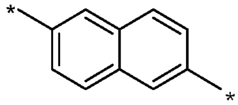
A10

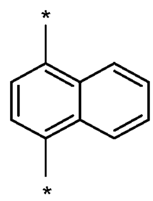
A11

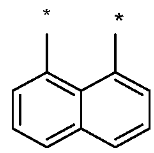
A12

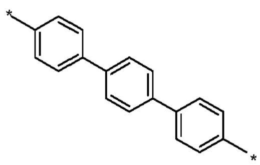
A13

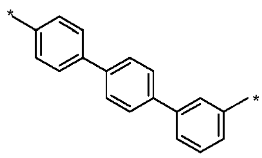
A14

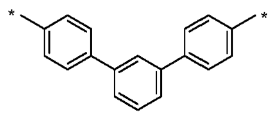
A15

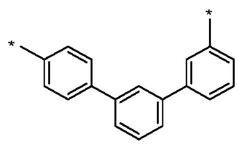
A16

wherein the asterisk symbol "*" represents the binding positions of L.

**8.** The organic electronic device (100) of any of the claims 1 to 7, whereby at least one of $R^1$ to $R^4$ or Ar may be selected from pyridine, quinolone, isoquinolone, indole, acridine, benzoacridine, dibenzoacridines, phenanthrodine, carbazole, indole, benzoindole, pyrimidine, pyrazine, quinozoline, pyrazole, quinoxaline, phenazine, naphthyridine, phananthrodine, azacarbazole, benzimidazole, benzooxazole, benzothiazole, nezotriazole, benzooxadiazole, benzothiadiazole, benzothiphene, benzofurane, dibenzofurane, dibenzothiophene, naphthofurane, naphthothiophene, phenanthroline.

**9.** The organic electronic device (100) of any of the claims 1 to 8, whereby the organic semiconductor layer (160) comprises a redox n-dopant.

**10.** The organic electronic device (100) of any of the claims 1 to 8, whereby the organic semiconductor layer (160) is an electron-transport layer.

**11.** The organic electronic device (100) of any of the claims 1 to 8, whereby the organic semiconductor layer (160) is a charge-generation layer.

**12.** The organic electronic device (100) of any of the claims 1 to 11, whereby the organic electronic device (100) is an electroluminescent device, preferably an organic light emitting diode.

**13.** A display device comprising an organic electronic device (100) according to any of the claims 1 to 12.

**14.** A $C_s$-symmetric compound of Formula (1):

5

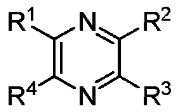

(1)

wherein

$R^1$-$R^4$ are independently selected from a substituted or unsubstituted $C_6$-$C_{36}$ aryl, and a substituted or unsubstituted $C_3$-$C_{36}$ heteroaryl;
wherein at least one of the $R^1$-$R^4$ is selected from $C_3$-$C_{36}$ heteroaryl group or heteroarylene group which is directly attached to the pyrazine ring in formula 1 and at least three of $R^1$-$R^4$ are different to each other;
wherein, if substituent(s) are present in $R^1$, $R^2$, $R^3$, and $R^4$, the substituent(s) are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, (C=O)$N(R)_2$, $Si(R)_3$, (S=O)R, and

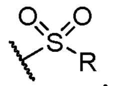
,

whereby
Y is O or S; and
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;
wherein two of the $R^1$-$R^4$ are phenyl or naphthenyl;
wherein at least one of the $R^1$ to $R^4$ is selected from formula (2):

$$-L-(Ar)_n \qquad (2)$$

whereby
L is substituted or unsubstituted $C_6$-$C_{36}$ arylene
Ar is selected from a substituted or unsubstituted $C_6$-$C_{36}$ aryl, and a substituted or unsubstituted $C_3$-$C_{36}$ heteroaryl;
n is 1 to 5;
two or more groups Ar may be identical to or different from each other;
and wherein the following compounds 1-4 are excluded:

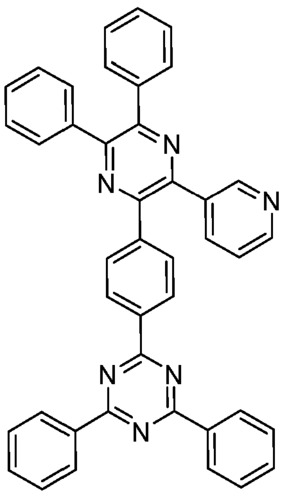

**1** ,

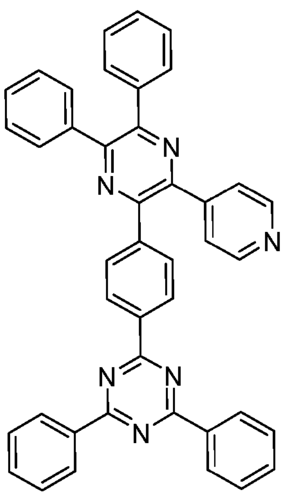

2 ,

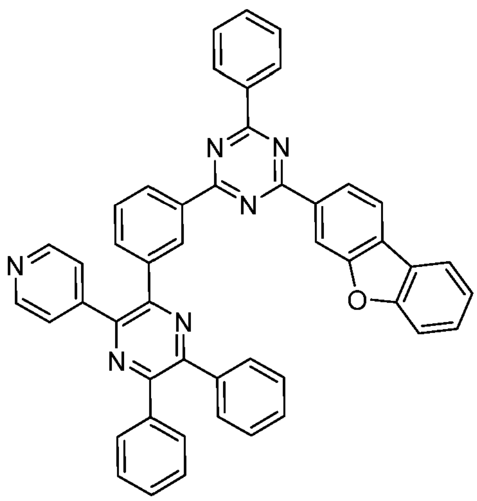

**3** ,

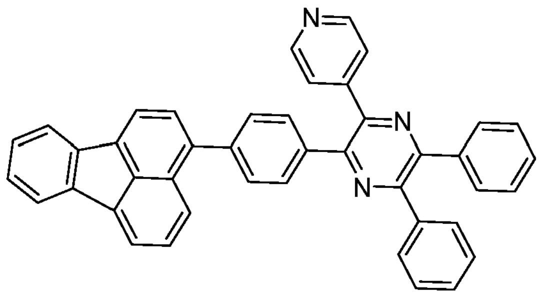

**4** .

**Patentansprüche**

1. Organische elektronische Vorrichtung (100), umfassend eine Anode (120), eine Kathode (190), mindestens eine photoaktive Schicht (125) und mindestens eine organische Halbleiterschicht (160), wobei die mindestens eine organische Halbleiterschicht (160) zwischen der mindestens einen photoaktiven Schicht (125) und der Kathode (190) angeordnet ist; und wobei die mindestens eine organische Halbleiterschicht (160) eine $C_s$-symmetrische Verbindung der Formel (1) umfasst:

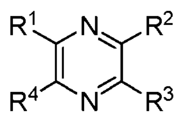

(1)

wobei

$R^1$ bis $R^4$ unabhängig aus einem substituierten oder unsubstituierten $C_6$- bis $C_{36}$-Aryl und einem substituierten oder unsubstituierten $C_3$- bis $C_{36}$-Heteroaryl ausgewählt sind;
wobei mindestens eine der $R^1$ bis $R^4$ aus einer $C_3$- bis $C_{36}$-Heteroaryl-Gruppe oder Heteroarylen-Gruppe ausgewählt ist, die direkt an den Pyrazin-Ring in Formel 1 gebunden ist und mindestens drei der $R^1$ bis $R^4$ voneinander verschieden sind;
wobei, wenn ein oder mehrere Substituenten in $R^1$, $R^2$, $R^3$ und $R^4$ vorhanden sind, der oder die Substituenten unabhängig ausgewählt sind aus den Gruppen, bestehend aus $C_6$- bis $C_{18}$-Aryl, $C_3$- bis $C_{20}$-Heteroaryl, D, F, CN, $C_1$- bis $C_{16}$-Alkyl, $C_1$- bis $C_{16}$-Alkoxy, $PY(R)_2$, OR, SR, (C=O)R, $(C=O)N(R)_2$, $Si(R)_3$, (S=O)R und

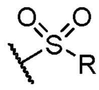,

wobei
Y für O oder S steht; und
R unabhängig ausgewählt ist aus linearem $C_1$- bis $C_{20}$-Alkyl, $C_1$- bis $C_{20}$-Alkoxy, $C_1$- bis $C_{20}$-Thioalkyl, verzweigtem $C_3$- bis $C_{20}$-Alkyl, zyklischem $C_3$- bis $C_{20}$-Alkyl, verzweigtem $C_3$- bis $C_{20}$-Alkoxy, zyklischem $C_3$- bis $C_{20}$-Alkoxy, verzweigtem $C_3$- bis $C_{20}$-Thioalkyl, zyklischem $C_3$- bis $C_{20}$-Thioalkyl, $C_6$- bis $C_{20}$-Aryl und $C_3$- bis $C_{20}$-Heteroaryl;
wobei zwei der $R^1$ bis $R^4$ Phenyl oder Naphthenyl sind; und wobei die folgenden Verbindungen 1 bis 4 ausgeschlossen sind:

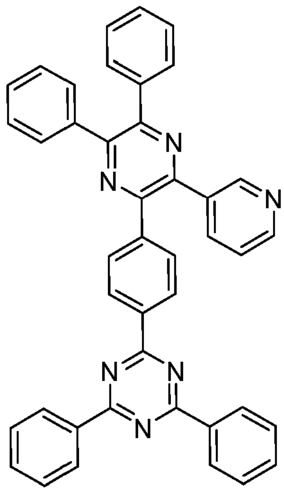

**1** ,

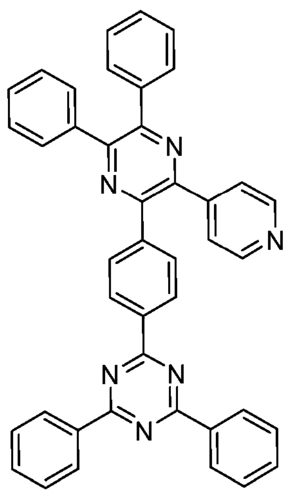

2 ,

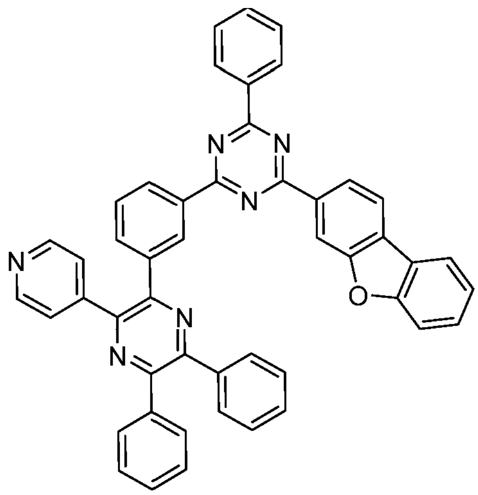

**3** ,

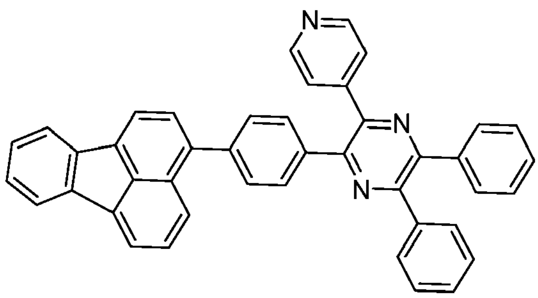

**4** .

2. Organische elektronische Vorrichtung (100) nach Anspruch 1, wobei die $R^1$ bis $R^4$, die kein substituiertes oder unsubstituiertes $C_3$- bis $C_{36}$-Heteroaryl oder Heteroarylen sind, das in Formel (1) über eine Einfachbindung direkt an den Pyrazin-Ring gebunden ist, ausgewählt sind aus substituiertem oder unsubstituiertem $C_6$- bis $C_{20}$-Aryl und einem substituierten oder unsubstituierten $C_3$- bis $C_{18}$-Heteroaryl.

3. Organische elektronische Vorrichtung (100) nach Anspruch 1 oder 2, wobei die mindestens eine der $R^1$ bis $R^4$, die $C_3$- bis $C_{36}$-Heteroaryl-Gruppe oder Heteroarylen-Gruppe ist, die durch eine Einfachbindung direkt an den Pyrazin-Ring in Formel (1) gebunden ist, ein substituiertes oder unsubstituiertes $C_3$- bis $C_{18}$-Heteroaryl oder Heteroarylen ist.

4. Organische elektronische Vorrichtung (100) nach einem der Ansprüche 1 bis 3, wobei die Verbindung ein Dipolmoment von ≥0,4 D aufweist

5. Organische elektronische Vorrichtung (100) nach einem der Ansprüche 1 bis 4, wobei zwei der $R^1$ bis $R^4$ Phenyl oder

Naphthenyl sind und in ortho-Position zueinander sind.

**6.** Organische elektronische Vorrichtung (100) nach einem der Ansprüche 1 bis 5, wobei mindestens eine der $R^1$ bis $R^4$ aus der Formel (2) ausgewählt ist:

$$-L-(Ar)_n \qquad (2)$$

wobei

L für substituiertes oder unsubstituiertes $C_6$- bis $C_{36}$-Arylen steht,
Ar aus einem substituierten oder unsubstituierten $C_6$- bis $C_{36}$-Aryl und einem substituierten oder unsubstituierten $C_3$- bis $C_{36}$-Heteroaryl ausgewählt ist,
n für 1 bis 5 steht;
zwei oder mehr Gruppen Ar identisch oder voneinander verschieden sein können.

**7.** Organische elektronische Vorrichtung (100) nach einem der Ansprüche 1 bis 6, wobei L aus einer der folgenden Reste A1 bis A16 ausgewählt ist:

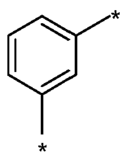
A1

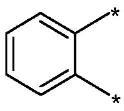
A2

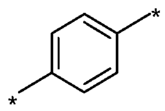
A3

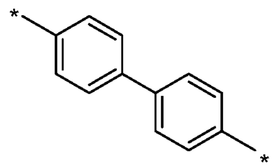
A4

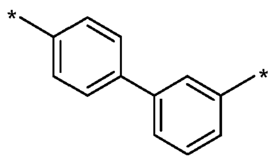
A5

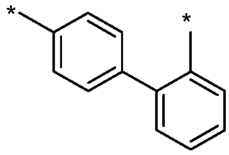
A6

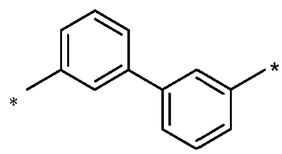
A7

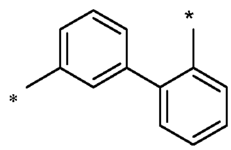
A8

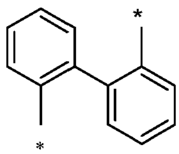
A9

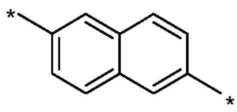
A10

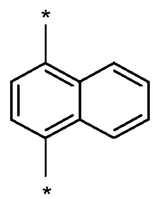
A11

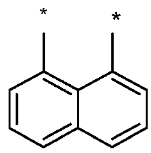
A12

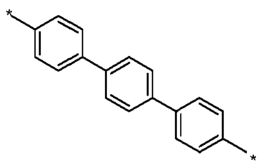
A13

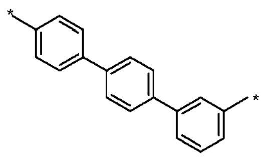
A14

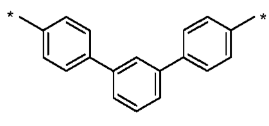
A15

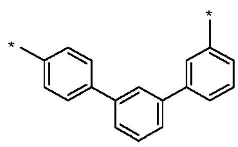
A16

wobei das Sternchensymbol "*" die Bindungspositionen von L repräsentiert.

**8.** Organische elektronische Vorrichtung (100) nach einem der Ansprüche 1 bis 7, wobei mindestens eine von $R^1$ bis $R^4$ oder Ar ausgewählt sein kann aus Pyridin, Chinolon, Isochinolon, Indol, Acridin, Benzoacridin, Dibenzoacridinen, Phenanthrodin, Carbazol, Indol, Benzoindol, Pyrimidin, Pyrazin, Chinozolin, Pyrazol, Chinoxalin, Phenazin, Naphthyridin, Phananthrodin, Azacarbazol, Benzimidazol, Benzooxazol, Benzothiazol, Nezotriazol, Benzooxadiazol, Benzothiadiazol, Benzothiophen, Benzofuran, Dibenzofuran, Dibenzothiophen, Naphthofuran, Naphthothiophen, Phenanthrolin.

**9.** Organische elektronische Vorrichtung (100) nach einem der Ansprüche 1 bis 8, wobei die organische Halbleiterschicht (160) einen n-Redoxdotierstoff umfasst.

**10.** Organische elektronische Vorrichtung (100) nach einem der Ansprüche 1 bis 8, wobei die organische Halbleiterschicht (160) eine Elektronentransportschicht ist.

**11.** Organische elektronische Vorrichtung (100) nach einem der Ansprüche 1 bis 8, wobei die organische Halbleiterschicht (160) eine Ladungserzeugungsschicht ist.

**12.** Organische elektronische Vorrichtung (100) nach einem der Ansprüche 1 bis 11, wobei die organische elektronische Vorrichtung (100) eine elektrolumineszierende Vorrichtung ist, vorzugsweise eine organische Leuchtdiode.

**13.** Anzeigevorrichtung, die eine organische elektronische Vorrichtung (100) nach einem der Ansprüche 1 bis 12 umfasst.

**14.** $C_s$-symmetrische Verbindung der Formel (1):

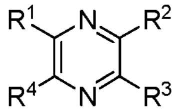

(1)

wobei

$R^1$ bis $R^4$ unabhängig aus einem substituierten oder unsubstituierten $C_6$- bis $C_{36}$-Aryl und einem substituierten oder unsubstituierten $C_3$- bis $C_{36}$-Heteroaryl ausgewählt sind;
wobei mindestens eine der $R^1$ bis $R^4$ ausgewählt ist aus einer $C_3$- bis $C_{36}$-Heteroaryl-Gruppe oder Heteroarylen-Gruppe, die direkt an den Pyrazin-Ring in Formel 1 gebunden ist und mindestens drei der $R^1$ bis $R^4$ voneinander verschieden sind;
wobei, wenn ein oder mehrere Substituenten in $R^1$, $R^2$, $R^3$ und $R^4$ vorhanden sind, der oder die Substituenten unabhängig ausgewählt sind aus den Gruppen, bestehend aus $C_6$- bis $C_{18}$-Aryl, $C_3$- bis $C_{20}$-Heteroaryl, D, F, CN, $C_1$- bis $C_{16}$-Alkyl, $C_1$- bis $C_{16}$-Alkoxy, $PY(R)_2$, OR, SR, (C=O)R, (C=O)N(R)$_2$, Si(R)$_3$, (S=O)R und

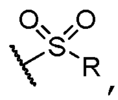,

wobei
Y für O oder S steht; und
R unabhängig ausgewählt ist aus linearem $C_1$- bis $C_{20}$-Alkyl, $C_1$- bis $C_{20}$-Alkoxy, $C_1$- bis $C_{20}$-Thioalkyl, verzweigtem $C_3$- bis $C_{20}$-Alkyl, zyklischem $C_3$- bis $C_{20}$-Alkyl, verzweigtem $C_3$- bis $C_{20}$-Alkoxy, zyklischem $C_3$- bis $C_{20}$-Alkoxy, verzweigtem $C_3$- bis $C_{20}$-Thioalkyl, zyklischem $C_3$- bis $C_{20}$-Thioalkyl, $C_6$- bis $C_{20}$-Aryl und $C_3$- bis $C_{20}$-Heteroaryl;
wobei zwei der $R^1$ bis $R^4$ Phenyl oder Naphthenyl sind; wobei mindestens eine der $R^1$ bis $R^4$ ausgewählt ist aus Formel (2):

$$-L-(Ar)_n \qquad (2)$$

wobei
L für substituiertes oder unsubstituiertes $C_6$- bis $C_{36}$-Arylen steht,
Ar aus einem substituierten oder unsubstituierten $C_6$- bis $C_{36}$-Aryl und einem substituierten oder unsubstituierten $C_3$- bis $C_{36}$-Heteroaryl ausgewählt ist,
n für 1 bis 5 steht;
zwei oder mehr Gruppen Ar identisch oder voneinander verschieden sein können;
und wobei die folgenden Verbindungen 1 bis 4 ausgeschlossen sind:

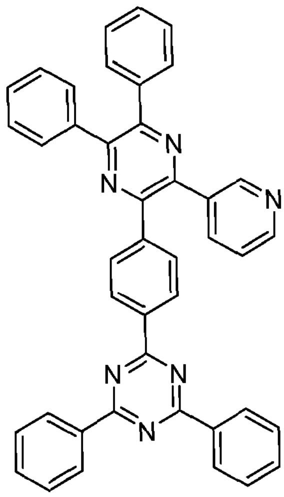

**1** ,

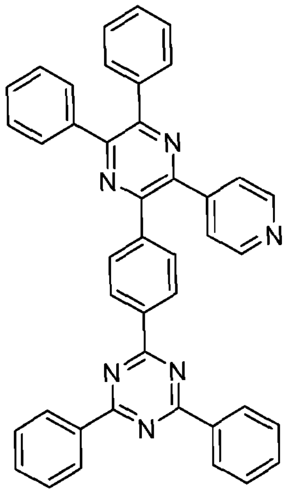

2 ,

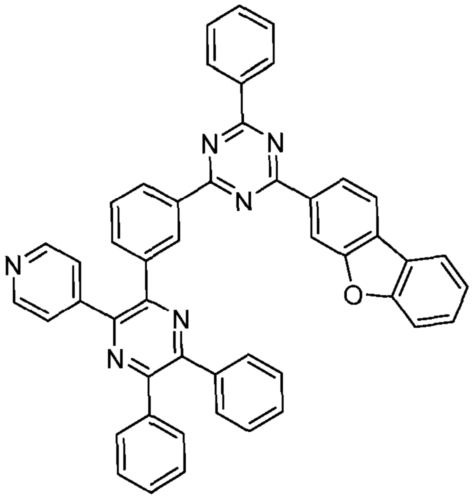

**3** ,

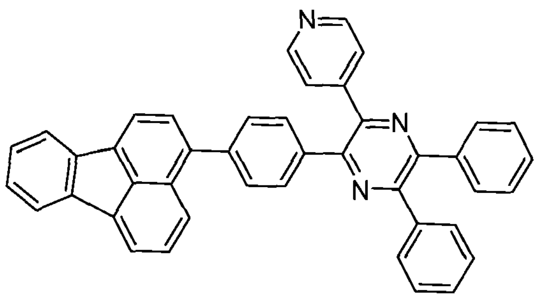

**4** .

## Revendications

**1.** . Dispositif électronique organique (100) comprenant une anode (120), une cathode (190), au moins une couche photoactive (125) et au moins une couche semi-conductrice organique (160), l'au moins une couche semi-conductrice organique (160) étant agencée entre l'au moins une couche photoactive (125) et la cathode (190) ; et l'au moins une couche semi-conductrice organique (160) comprenant un composé de formule (1) à symétrie $C_s$ :

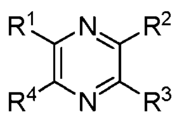

(1)

avec

$R^1$-$R^4$ étant indépendamment choisi parmi un aryle en $C_6$-$C_{36}$ substitué ou non substitué, et un hétéroaryle en

$C_3$-$C_{36}$ substitué ou non substitué ;
au moins l'un des $R^1$-$R^4$ étant choisi parmi un groupe hétéroaryle ou un groupe hétéroarylène en $C_3$-$C_{36}$ qui est directement fixé au cycle pyrazine dans la formule 1 et au moins trois parmi $R^1$-$R^4$ étant différents les uns des autres ;
si un ou des substituants sont présents dans $R^1$, $R^2$, $R^3$, et $R^4$, le ou les substituants étant indépendamment choisis parmi les groupes consistant en des groupes aryle en $C_6$-$C_{18}$, hétéroaryle en $C_3$-$C_{20}$, D, F, CN, alkyle en $C_1$-$C_{16}$, alcoxy en $C_1$-$C_{16}$, PY(R)2, OR, SR, (C=O)R, (C=O)N(R)2, Si(R)3, (S=O)R, et

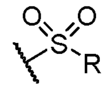

,

avec
Y étant O ou S ; et
R étant choisi indépendamment parmi des groupes alkyle linéaire en $C_1$-$C_{20}$, alcoxy en $C_1$-$C_{20}$, thioalkyle en $C_1$-$C_{20}$, alkyle ramifié en $C_3$-$C_{20}$, alkyle cyclique en $C_3$-$C_{20}$, alcoxy ramifié en $C_3$-$C_{20}$, alcoxy cyclique en $C_3$-$C_{20}$, thioalkyle ramifié en $C_3$-$C_{20}$, thioalkyle cyclique en $C_3$-$C_{20}$, aryle en $C_6$-$C_{20}$ et hétéroaryle en $C_3$-$C_{20}$ ;
deux des $R^1$-$R^4$ étant des groupes phényle ou naphténylé ; et les composés 1-4 suivants étant exclus :

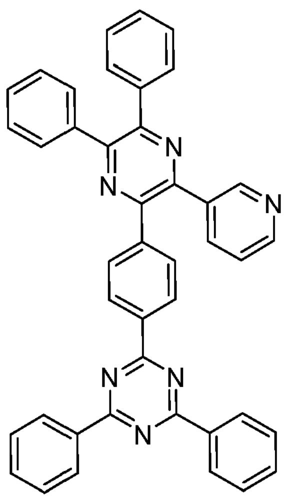

**1** ,

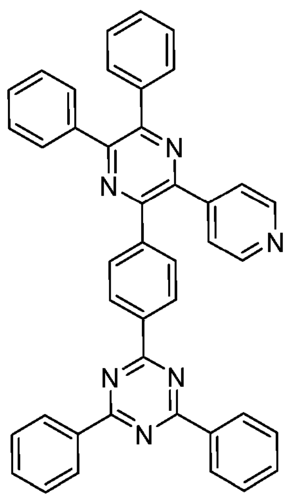

2 ,

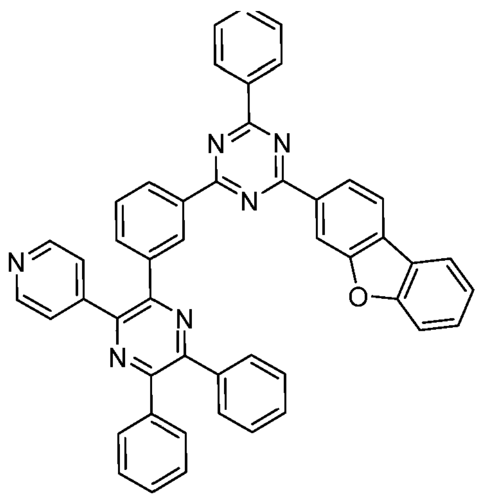

**3** ,

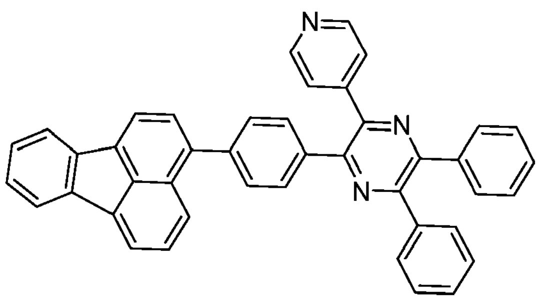

**4** .

2. . Dispositif électronique organique (100) selon la revendication 1, les $R^1$ à $R^4$ qui ne sont pas un hétéroaryle ou hétéroarylène en $C_3$-$C_{36}$ substitué ou non substitué, qui est directement fixé au cycle pyrazine dans la formule (1) par l'intermédiaire d'une simple liaison étant choisis parmi un aryle en $C_6$-$C_{20}$ substitué ou non substitué, et un hétéroaryle en $C_3$-$C_{18}$ substitué ou non substitué.

3. . Dispositif électronique organique (100) selon la revendication 1 ou 2, l'au moins un des $R^1$ à $R^4$ qui est un groupe hétéroaryle en $C_3$-$C_{36}$ ou un groupe hétéroarylène qui est directement fixé au cycle pyrazine dans la formule (1) par

l'intermédiaire d'une simple liaison étant un hétéroaryle ou hétéroarylène en $C_3$-$C_{18}$ substitué ou non substitué.

**4.** . Dispositif électronique organique (100) selon l'une quelconque des revendications 1 à 3, le composé ayant un moment dipolaire ≥ 0,4 D.

**5.** . Dispositif électronique organique (100) selon l'une quelconque des revendications 1 à 4, deux des $R^1$-$R^4$ étant des groupes phényle ou naphtényle et en position ortho l'un par rapport à l'autre.

**6.** . Dispositif électronique organique (100) selon l'une quelconque des revendications 1 à 5, au moins l'un des $R^1$-$R^4$ étant choisi parmi la formule (2) :

$$-L-(Ar)_n \qquad (2)$$

avec

L étant un arylène en $C_6$-$C_{36}$ substitué ou non substitué Ar étant choisi parmi un aryle en $C_6$-$C_{36}$ substitué ou non substitué, et un hétéroaryle en $C_3$-$C_{36}$ substitué ou non substitué,
n étant 1 à 5 ;
deux groupements Ar ou plus pouvant être identiques ou différents l'un de l'autre.

**7.** . Dispositif électronique organique (100) selon l'une quelconque des revendications 1 à 6, L étant choisi parmi l'un des groupements suivants A1 à A16 :

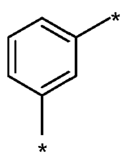
A1

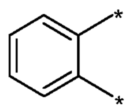
A2

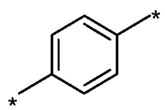
A3

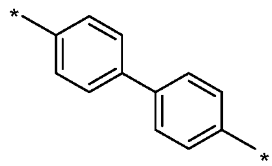
A4

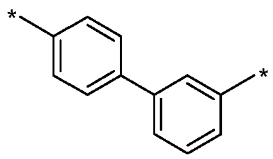
A5

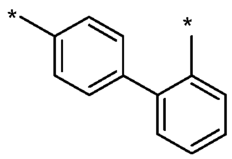
A6

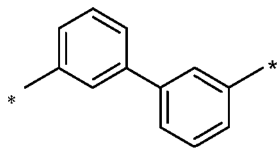
A7

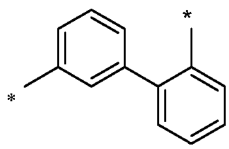
A8

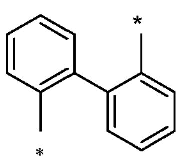
A9

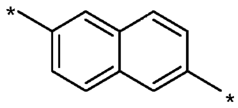
A10

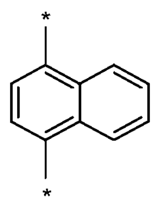
A11

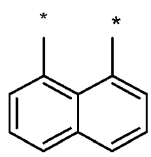
A12

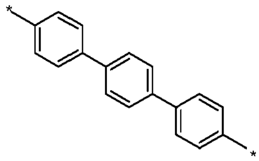
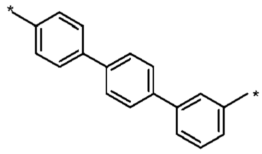
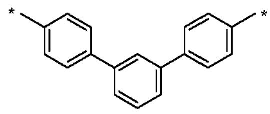
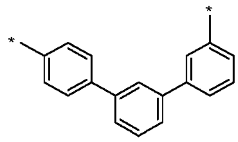

A13 A14 A15 A16

le symbole astérisque « * » représentant les positions de liaison de L.

**8.** . Dispositif électronique organique (100) selon l'une quelconque des revendications 1 à 7, au moins l'un parmi $R^1$ à $R^4$ ou Ar pouvant être choisi parmi la pyridine, la quinolone, l'isoquinolone, l'indole, l'acridine, benzoacridine, les dibenzoacridines, la phénanthrodine, le carbazole, l'indole, le benzoindole, la pyrimidine, la pyrazine, la quinozoline, le pyrazole, la quinoxaline, la phénazine, la naphtyridine, la phananthrodine, l'azacarbazole, le benzimidazole, le benzooxazole, le benzothiazole, le nézotriazole, le benzooxadiazole, le benzothiadiazole, le benzothiphène, le benzofuranne, le dibenzofuranne, le dibenzothiophène, le naphtofuranne, le naphtothiophène, la phénanthroline.

**9.** . Dispositif électronique organique (100) selon l'une quelconque des revendications 1 à 8, la couche semi-conductrice organique (160) comprenant un dopant n redox.

**10.** . Dispositif électronique organique (100) selon l'une quelconque des revendications 1 à 8, la couche semi-conductrice organique (160) étant une couche de transport d'électrons.

**11.** . Dispositif électronique organique (100) selon l'une quelconque des revendications 1 à 8, la couche semi-conductrice organique (160) étant une couche de génération de charge.

**12.** . Dispositif électronique organique (100) selon l'une quelconque des revendications 1 à 11, le dispositif électronique organique (100) étant un dispositif électroluminescent, de préférence une diode électroluminescente organique.

**13.** . Dispositif d'affichage comprenant un dispositif électronique organique (100) selon l'une quelconque des revendications 1 à 12.

**14.** . Composé de formule (1) à symétrie Cs :

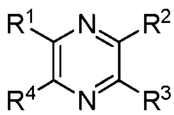

(1)

avec

$R^1$-$R^4$ étant indépendamment choisi parmi un aryle en $C_6$-$C_{36}$ substitué ou non substitué, et un hétéroaryle en $C_3$-$C_{36}$ substitué ou non substitué ;
au moins l'un des $R^1$-$R^4$ étant choisi parmi un groupe hétéroaryle ou un groupe hétéroarylène en $C_3$-$C_{36}$ qui est directement fixé au cycle pyrazine dans la formule 1 et au moins trois parmi $R^1$-$R^4$ étant différents les uns des autres ;
si un ou des substituants sont présents dans $R^1$, $R^2$, $R^3$, et $R^4$, le ou les substituants étant indépendamment choisis parmi les groupes consistant en des groupes aryle en $C_6$-$C_{18}$, hétéroaryle en $C_3$-$C_{20}$, D, F, CN, alkyle en $C_1$-$C_{16}$, alcoxy en $C_1$-$C_{16}$, PY(R)2, OR, SR, (C=O)R, (C=O)N(R)2, Si(R)3, (S=O)R, et

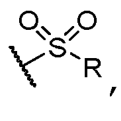

avec

Y étant O ou S ; et

R étant choisi indépendamment parmi des groupes alkyle linéaire en $C_1$-$C_{20}$, alcoxy en $C_1$-$C_{20}$, thioalkyle en $C_1$-$C_{20}$, alkyle ramifié en $C_3$-$C_{20}$, alkyle cyclique en $C_3$-$C_{20}$, alcoxy ramifié en $C_3$-$C_{20}$, alcoxy cyclique en $C_3$-$C_{20}$, thioalkyle ramifié en $C_3$-$C_{20}$, thioalkyle cyclique en $C_3$-$C_{20}$, aryle en $C_6$-$C_{20}$ et hétéroaryle en $C_3$-$C_{20}$ ;

deux des $R^1$-$R^4$ étant des groupes phényle ou naphtényle ; au moins l'un des $R^1$ à $R^4$ étant choisi parmi la formule (2) :

$$-L-(Ar)_n \qquad (2)$$

avec

L étant un arylène en $C_6$-$C_{36}$ substitué ou non substitué Ar étant choisi parmi un aryle en $C_6$-$C_{36}$ substitué ou non substitué, et un hétéroaryle en $C_3$-$C_{36}$ substitué ou non substitué,

n étant 1 à 5 ;

deux groupements Ar ou plus pouvant être identiques ou différents l'un de l'autre ;

et les composés 1-4 suivants étant exclus :

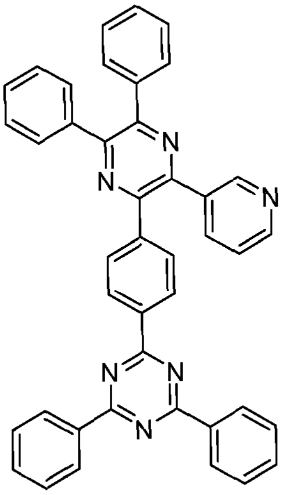

1 ,

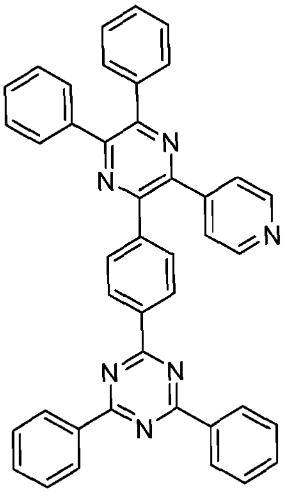

2 ,

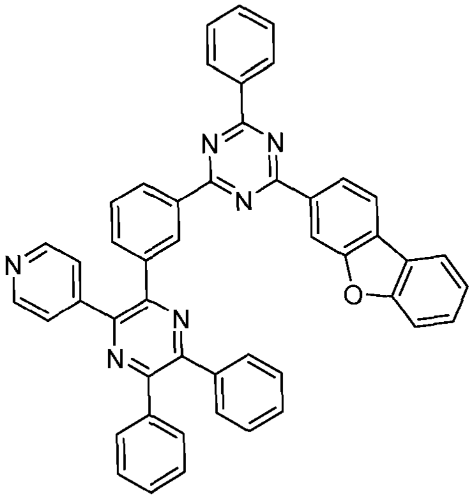

3 ,

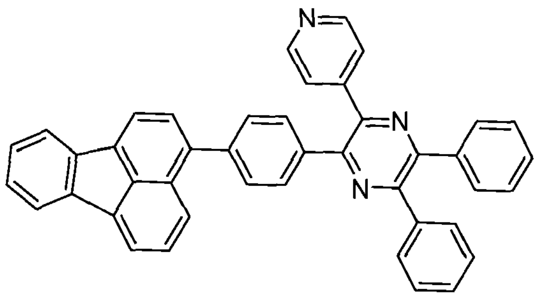

4 .

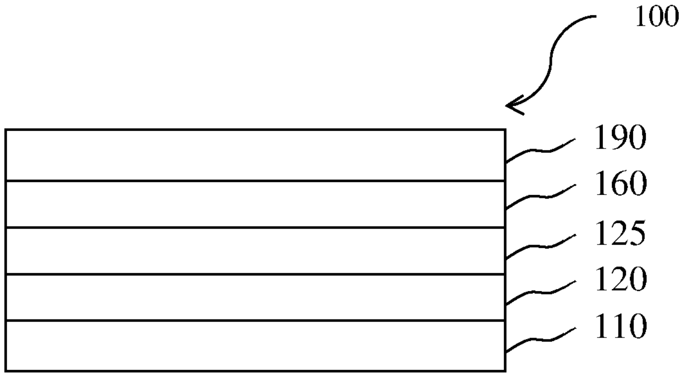


Fig.1

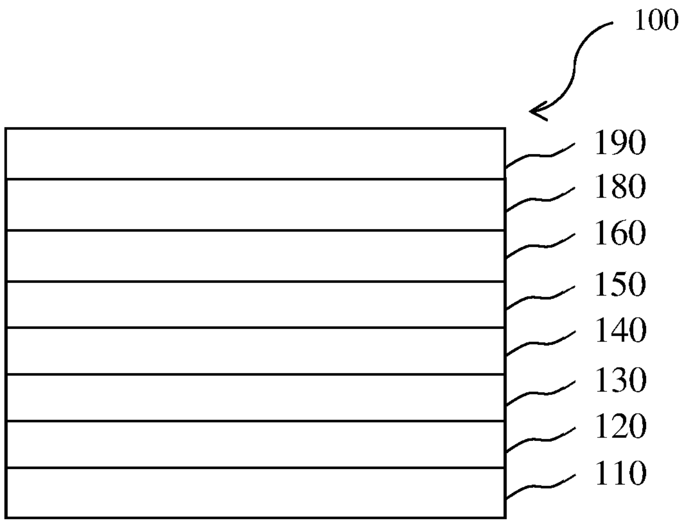


Fig.2

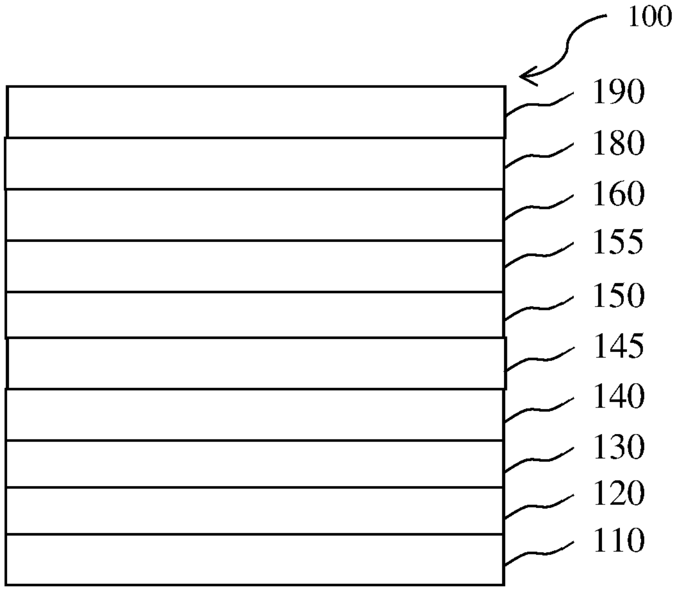


Fig.3

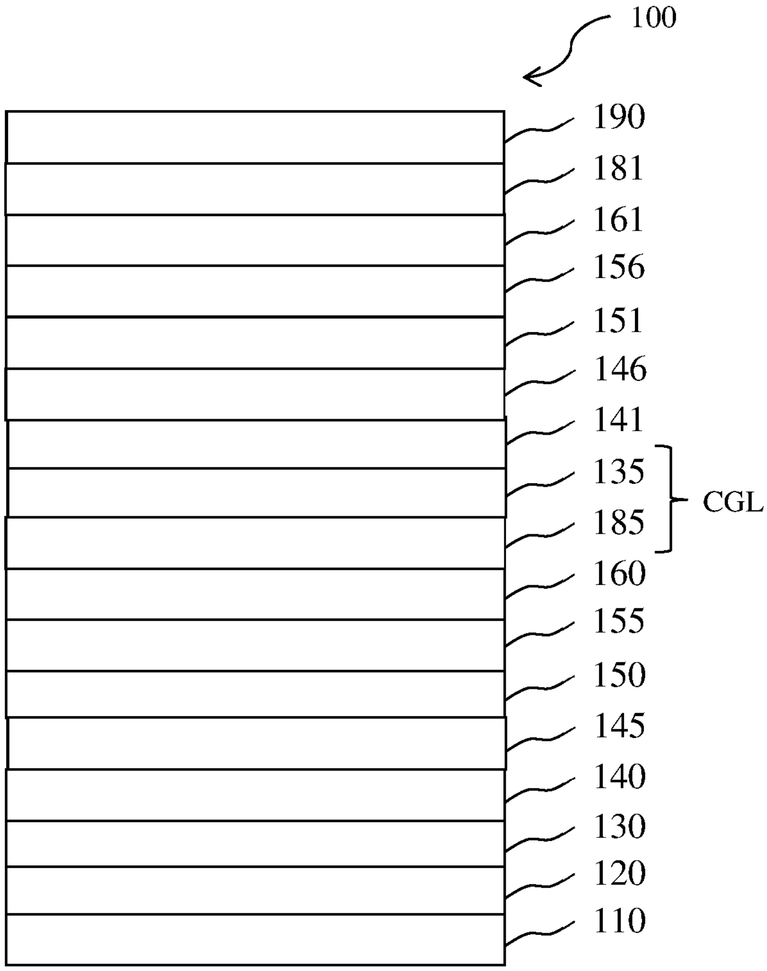
100
190
181
161
156
151
146
141
135
185
160
155
150
145
140
130
120
110
CGL

Fig.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 110229145 A
- KR 20160006629 A
- WO 2017145546 A1
- WO 2005086251 A
- EP 1837926 B1
- WO 2007107306 A
- WO 2007107356 A
- EP 2722908 A1
- EP 1970371 A1
- WO 2013079217 A1


### Non-patent literature cited in the description


- **YASUHIKO SHIROTA** ; **HIROSHI KAGEYAMA**. *Chem. Rev.*, 2007, vol. 107, 953-1010
- *CHEMICAL ABSTRACTS*, 1198399-61-9
- *CHEMICAL ABSTRACTS*, 1242056-42-3